(19) Europäisches Patentamt
European Patent Office
Office européen des brevets

(11) **EP 1 984 490 B1**

(12) **FASCICULE DE BREVET EUROPEEN**

(45) Date de publication et mention
de la délivrance du brevet:
**09.11.2016 Bulletin 2016/45**

(21) Numéro de dépôt: **07730876.5**

(22) Date de dépôt: **26.01.2007**

(51) Int Cl.:
*A61K 35/12* (2006.01)     *C12N 5/077* (2010.01)

(86) Numéro de dépôt international:
**PCT/FR2007/000158**

(87) Numéro de publication internationale:
**WO 2007/085745 (02.08.2007 Gazette 2007/31)**

(54) **PROCEDE DE CULTURE DE CELLULES ISSUES DU TISSU ADIPEUX ET LEURS APPLICATIONS**

VERFAHREN ZUR KULTIVIERUNG VON ZELLEN AUS FETTGEWEBE UND IHRE VERWENDUNG

METHOD FOR CULTURING CELLS DERIVED FROM THE ADIPOSE TISSUE AND USES THEREOF

(84) Etats contractants désignés:
**AT BE BG CH CY CZ DE DK EE ES FI FR GB GR
HU IE IS IT LI LT LU LV MC NL PL PT RO SE SI
SK TR**

(30) Priorité: **26.01.2006 FR 0600710**

(43) Date de publication de la demande:
**29.10.2008 Bulletin 2008/44**

(73) Titulaire: **Centre National de la Recherche
Scientifique
75016 Paris (FR)**

(72) Inventeurs:
• **CASTEILLA, Louis**
  **F-37150 Escalquens (FR)**
• **PLANAT-BENARD, Valérie**
  **F-31120 Lacroix Falgarde (FR)**
• **PENICAUD, Luc**
  **F-31400 Toulouse (FR)**
• **JOFFRE-CHANUT, Carine**
  **N52PE London (GB)**

(74) Mandataire: **Gevers & Orès
41 avenue de Friedland
75008 Paris (FR)**

(56) Documents cités:
**WO-A-95/20042          WO-A-02/055678
WO-A-2005/042730**

• **PLANAT-BENARD V ET AL: "Spontaneous
cardiomyocyte differentiation from adipose
tissue stroma cells." CIRCULATION RESEARCH,
vol. 94, no. 2, 6 février 2004 (2004-02-06), pages
223-229, XP002400832 ISSN: 0009-7330**
• **FORTIER LISA A: "Stem cells: classifications,
controversies, and clinical applications."
VETERINARY SURGERY : VS : THE OFFICIAL
JOURNAL OF THE AMERICAN COLLEGE OF
VETERINARY SURGEONS 2005 SEP-OCT, vol.
34, no. 5, septembre 2005 (2005-09), pages
415-423, XP002411799 ISSN: 0161-3499**
• **ZHANG M ET AL: "Cardiomyocyte grafting for
cardiac repair: graft cell death and anti-death
strategies", JOURNAL OF MOLECULAR AND
CELLULAR CARDIOLOGY, ACADEMIC PRESS,
GB, vol. 33, no. 5, 1 May 2001 (2001-05-01), pages
907-921, XP002411879, ISSN: 0022-2828, DOI:
10.1006/JMCC.2001.1367**

SEQUENCE LISTING

<110> CENTRE NATIONAL DE LA RECHERCHE SCIENTIFIQUE CASTEILLA, Louis PLANAT-BENARD, Valérie PENICAUD, Luc CHANUT, Carine

<120> PROCEDE DE CULTURE DE CELLULES ISSUES DU TISSU ADIPEUX ET LEURS APPLICATIONS

<130> BLOcp/131PCT

<160> 12

<170> PatentIn version 3.3

<210> 1
<211> 25
<212> DNA
<213> Artificial

<220>
<223> Amorce sens MEF2C

<400> 1
agatacccac aacacaccac gcgcc        25

<210> 2
<211> 25
<212> DNA
<213> Artificial

<220>
<223> Amorce antisens MEF2C

<400> 2
atccttcaga gagtcgcatg cgctt          25

<210> 3
<211> 20
<212> DNA
<213> Artificial

<220>
<223> Amorce sens Oct-3/4

<400> 3
tcagcttggg ctagagaagg          20

<210> 4
<211> 20
<212> DNA
<213> Artificial

<220>
<223> Amorce antisens Oct-3/4

<400> 4
tgacgggaac agagggaaag          20

<210> 5
<211> 20
<212> DNA
<213> Artificial

<220>
<223> Amorce sens Brachury

<400> 5
gacttcgtga cggctgacaa          20

<210> 6
<211> 19
<212> DNA
<213> Artificial

<220>
<223> Amorce antisens Brachury

<400> 6
cgagtctggg tggatgtag          19

<210> 7
<211> 23
<212> DNA
<213> Artificial

<220>
<223> Amorce sens Isletl

<400> 7
catcgagtgt ttccgctgtg tag          23

```
<210> 8
<211> 24
<212> DNA
<213> Artificial

<220>
<223> Amorce antisens Isletl

<400> 8
gtggtcttct ccggctgctt gtgg          24


<210> 9
<211> 21
<212> DNA
<213> Artificial

<220>
<223> Amorce sens GFP

<400> 9
gggcacaagc tggagtacaa c          21


<210> 10
<211> 21
<212> DNA
<213> Artificial

<220>
<223> Amorce antisens GFP

<400> 10
tcaccttgat gccgttcttc t          21


<210> 11
<211> 23
<212> DNA
<213> Artificial

<220>
<223> Amorce sens 36B4

<400> 11
agtcggagga atcagatgag gat          23


<210> 12
<211> 20
<212> DNA
<213> Artificial

<220>
<223> Amorce antisens 36B4

<400> 12
ggctgacttg gttgctttgg          20
```

**Description**

La présente invention est relative à un procédé de culture de cellules issues du tissu adipeux et notamment de la fraction stroma-vasculaire (FSV ou SVF pour *stromal vascular fraction*) pour induire la formation de cardiomyocytes.

La présente invention a également pour objet l'utilisation des cellules obtenues par le procédé de culture tel que défini ci-dessus pour reconstituer une zone cardiaque ischémiée, notamment après infarctus, ainsi qu'à une composition pharmaceutique contenant lesdites cellules.

L'insuffisance cardiaque se développe, dans de nombreux cas, à la suite d'un accident ischémique (infarctus du myocarde) et est associée à une perte importante de cardiomyocytes et à une ischémie, qui n'est pas contrebalancée par le renouvellement normal des cardiomyocytes différenciés par le coeur.

Les moyens existants pour reconstituer les cardiomyocytes sont essentiellement basés sur des stratégies incluant une thérapie cellulaire, pour améliorer la performance contractile défectueuse et la fonction cardiaque (Wollert et al., Circulation, 2005, 112, 2, 151-153).

Ils comprennent essentiellement :

- la transplantation de myoblastes autologues ou de cellules satellites, qui améliorent la fonction cardiaque, même si ces cellules conservent leurs caractéristiques de cellules musculaires dans le coeur ischémique. Néanmoins, l'absence apparente de connexions entre les myoblastes greffés et les cardiomyocytes résidents exclut une contribution synchronisée des cellules greffées à la fonction de la pompe systolique.

- le concept de plasticité cellulaire a ouvert de nouvelles perspectives dans le domaine de l'insuffisance cardiaque et a conduit à utiliser différentes cellules dans l'espoir que ces cellules ectopiques puissent se transdifférencier en cardiomyocytes, lorsqu'elles sont placées dans un contexte *in vivo* adapté. En fait, des études initiales ont suggéré que les cellules souches mésenchymateuses de la moelle osseuse et les cellules hématopoïétiques peuvent se transdifférencier en cardiomyocytes ; toutefois, ce phénomène est actuellement discuté ainsi que le concept de plasticité cellulaire.

- une autre voie, moins compliquée, a été préconisée et propose de mettre en oeuvre des cellules engagées dans le processus de transformation en cardiomyocytes, telles que les cellules souches cardiomyocytaires résidentes, les cellules foetales, les cellules embryonnaires ou d'autres cellules telles que des cellules mésenchymateuses de la moelle osseuse ou des cellules endothéliales (Kehat et al., J. Clin. Invest., 2001, 108, 407-444 ; Muller et al., FASEB J., 2000, 14, 2540-2558 ; Toma et al., Circulation, 2002, 105, 93-98 ; Liechty et al., Nat. Medecine, 2000, 11, 1282-1286 ; Conderelli et al., PNAS, 2001, 98, 10733-10738 ; Wang et al., J. Thorac. Cardiovasc. Surg 2001, 122, 699-705 ; Jackson et al., J. Clin. Invest. 2001, 107, 1395-1402). Deux hypothèses ont été émises pour expliquer ces résultats : des cellules proches de la totipotence des cellules embryonnaires persisteraient dans des tissus de l'adulte (cerveau, muscle...) et seraient capables de se différencier en différents types cellulaires, ou bien des cellules souches spécialisées de ces tissus posséderaient une grande plasticité et seraient capables de se dé-différencier ou d'être reprogrammées (transdifférenciation). Ces résultats ont des conséquences importantes pour le traitement des déficits musculaires fonctionnels (myopathies et cardiomyopathies) et des maladies liées à une dégénérescence musculaire (infarctus du myocarde). Cependant, en pratique, la reconstruction effective des tissus musculaires cardiaques à partir des cellules précitées est difficilement réalisable en raison des difficultés techniques de prélèvement et des faibles quantités de tissus disponibles. A ces difficultés techniques, s'ajoutent également des problèmes éthiques liés à l'utilisation de tissus embryonnaires (M/S, 2004, 6-7, 20, 651-661).

Dans ce contexte, il existe un réel besoin de nouveaux moyens et notamment de nouvelles sources de cellules aptes à effectivement reconstruire le myocarde, qui soient efficaces et plus simples à mettre en oeuvre que les moyens existants.

Par conséquent, les Inventeurs se sont donné pour but de pourvoir à des cellules aptes à reconstituer les tissus musculaires cardiaques, de façon durable, lesquelles cellules sont isolées à partir de tissus faciles à prélever et disponibles en quantités importantes.

Les Inventeurs, dans le cadre de leurs recherches précédentes ont trouvé (Demande WO 02/055678) qu'il existait une différenciation spontanée de cellules dérivées du tissu adipeux, et plus particulièrement des cellules de la fraction du stroma-vasculaire (FSV), en cardiomyocytes fonctionnels, lorsque ces cellules dérivées du tissu adipeux sont mises en culture dans un milieu contenant de la méthylcellulose (Demande Internationale PCT WO 02/055678 ; Planat et al., Cire. Res., 2004, 94, 223-229).

Ainsi, la Demande Internationale PCT WO 02/055678 préconise pour obtenir une différenciation de cellules de la FSV en cellules cardiogéniques, une étape de préparation de la FSV, une étape de tri cellulaire (sélection) comprenant la culture des cellules sur milieu semi-solide (méthylcellulose) et/ou la purification des cellules par séparation physique et/ou immunosélection (anticorps) et une étape dite d'expansion des cellules en milieu liquide DMEM-F12. Toutefois, il s'avère que la culture en milieu liquide DMEM-F12 ne permet en fait que le maintien des cellules cardiomyogéniques et non leur expansion (Planat-Bénard et al., Circ. Res., 2004, 94, 223-229).

En effet, l'Article aux noms de Planat-Bénard et al. précité montre que les cellules de la FSV sont capables de se différencier spontanément en cardiomyocytes lorsqu'elles sont mises en culture dans un milieu contenant de la méthylcellulose. Il est en outre précisé que ces cellules peuvent être entretenues plusieurs mois sur méthylcellulose ou dans un milieu de culture liquide DMEM-F12.

Ainsi, cet Article montre que l'on peut obtenir, de manière spontanée, à partir de la FSV, des cellules de type cardiomyocyte en culture primaire, lorsque l'on utilise comme milieu de culture, un milieu semi-solide à base de méthylcellulose. Par contre, lorsque l'on utilise le milieu liquide DMEM-F12, on observe très peu de cellules contractiles.

Ainsi, aussi bien le procédé décrit dans la Demande PCT Internationale WO 02/055678 que celui préconisé dans l'Article aux noms de Planat-Bénard et al. précité, bien que permettant effectivement d'obtenir des cardiomyocytes, ne fournissent pas un bon rendement de différenciation. En effet, la fréquence d'obtention de cardiomyocytes aussi bien dans le procédé décrit dans la Demande PCT Internationale WO 02/055678 que dans l'Article aux noms de Planat-Bénard et al. est très faible ; ceci est notamment dû à l'hétérogénéité des cellules SVF et à la variabilité de leur préparation, qui ne permettent pas d'obtenir, dans les conditions de culture standards précédemment décrites, un bon rendement de différenciation de ces cellules en cellules cardiaques.

Dans la mesure où dans les conditions précitées, cette différenciation est rare, les Inventeurs ont maintenant mis au point une méthode *d'expansion in vitro* présentant un très bon rendement en cellules cardiomyogéniques, à partir de tissu adipeux et plus particulièrement à partir de la fraction stroma-vasculaire.

Ainsi la Demande en instance s'est donné pour but d'obtenir des cellules cardiaques, à partir de FSV, avec un rendement élevé, en vue de leur utilisation thérapeutique.

Ils ont trouvé, de manière inattendue, que les cellules ainsi obtenues survivent, peuvent être greffées et se différencient en cardiomyocytes après leur implantation dans un modèle animal (souris présentant une ischémie aiguë) en comparaison avec des cellules du tissu adipeux en culture, non transformées en cardiomyocytes.

Au sens de la présente invention, on entend par :

- cellules cardiomyogéniques, toute cellule capable de se différencier en cellule de type cardiaque, y compris les progéniteurs cardiaques (cellules précurseurs présentes dans le coeur);
- cardiomyocytes, les cardiomyocytes contractiles, de forme cylindrique présentant des connexions avec les cellules myocardiques adjacentes, pour former un réseau tridimensionnel complexe;
- cellules de type cardiaque (ou à phénotype cardiaque), des cellules exprimant des protéines cardiaques (troponine T) ; il s'agit de cellules en cours de différenciation cardiaque ; elles se distinguent des cellules cardiomyogéniques proprement dites qui sont à potentiel cardiaque, c'est-à-dire aptes à s'engager dans une voie de différenciation cardiaque ;
- expansion cellulaire : on considère qu'il y a expansion cellulaire lorsque quelques cellules d'une culture cellulaire sont prélevées et ensemencées dans un nouveau milieu de culture, puis prolifèrent. Lorsque cette opération n'est réalisée qu'une seule fois, on a un seul passage. Lorsque l'on réalise cette opération plusieurs fois (ensemencement et division cellulaire), on effectue ainsi des passages successifs.
- maintien cellulaire : culture cellulaire sans passage.

Ci-après, l'expression cellules cardiaques (ou encore cellules cardiogéniques) inclut à la fois les cellules cardiomyogéniques, les progéniteurs cardiaques, les cellules de type cardiaque et les cardiomyocytes.

Le tissu adipeux existe sous différentes formes chez les mammifères : le tissu adipeux blanc extramédullaire qui représente le principal organe de réserve de l'organisme, le tissu adipeux blanc médullaire dont le rôle exact n'est pas connu et le tissu adipeux brun thermogénique.

Du fait de son potentiel d'expansion important qui persiste tout au long de la vie de l'individu, le tissu adipeux blanc de l'adulte constitue une source de cellules abondantes et faciles à obtenir.

Ce tissu adipeux blanc est constitué par deux fractions cellulaires :

- une fraction adipocytaire qui représente 30% à 60% des cellules du tissu adipeux et est caractérisée par l'accumulation de triglycérides (fraction de cellules flottantes). Cette fraction est composée en grande majorité (99 %) d'adipocytes différenciés et de quelques macrophages contaminants, riches en gouttelettes lipidiques, et
- une fraction non-adipocytaire, appelée fraction stroma-vasculaire (FSV), comprenant quelques cellules sanguines, quelques cellules endothéliales matures (cellules de l'endothélium microvasculaire : CD31$^+$, CD144$^+$), des péricytes, des fibroblastes et des cellule souches pluripotentes.

Il a été montré que la fraction stroma-vasculaire, classiquement utilisée pour étudier la différenciation des préadipocytes en adipocytes matures, était une source de cellules pluripotentes comprenant outre des progéniteurs adipocytaires (préadipocytes), des progéniteurs endothéliaux, hématopoïétiques et neurogéniques, ainsi que des cellules souches mésenchymateuses capables de se différencier en lignées ostéogéniques, chondrogéniques et myogéniques (Planat-Bénard V. et al., Circulation, 2004, 109, 656-663 ; Zuk PA. et al., Mol. Biol. Cell, 2002, 13, 4279-95 ; Erickson GR. et al., Biochem. Biophys. Res. Commun., 2002, 290, 763-9 ; Cousin B. et al., Biochem. Biophys. Res. Commun., 2003, 301, 1016-22 ; Safford KM. et al., Biochem. Biophys. Res. Commun., 2002, 294, 371-9; Demande Internationale PCT WO 02/055678 et Demande Américaine US 2003/0082152).

Ces deux fractions cellulaires peuvent être séparées par leur différence de densité, selon des procédés tels que ceux décrits par Björntorp et al. (J. Lipid. Res., 1978, 19, 316-24).

Les Inventeurs ont maintenant mis au point des conditions de culture qui augmentent de manière significative le nombre de cellules SVF capables de se transformer (ou de se transdifférencier) en cellules à phénotype cardiaque :

- soit directement à partir de la SVF brute fraîchement préparée, à l'aide d'au moins un marqueur phénotypique, qui permet de sélectionner directement une population d'intérêt (tri CMH1 négatif, par exemple), suivie d'une culture dans un milieu liquide convenable,
- soit après culture primaire de la SVF brute fraîchement préparée dans un milieu semi solide, suivie d'une culture secondaire des cellules obtenues dans un milieu liquide convenable ; dans ce cas, un tri cellulaire peut en outre être effectué avant la culture secondaire.

La présence de cellules cardiaques dans le tissu adipeux est surprenante et les signaux impliqués dans la transformation des cellules quiescentes résidentes en cellules présentant un phénotype cardiaque contractile n'étaient pas connus jusqu'à présent.

Le TGF$\beta$ et $H_2O_2$ étaient décrits comme efficaces à promouvoir la différenciation des cardiomyocytes néonataux et des cellules souches embryonnaires, mais ne permettaient pas l'induction d'une telle transformation à partir de cellules dérivées du tissu adipeux.

Parmi les facteurs testés (IL3, IL6, SCF, BMP2, TGF$\beta$, 5-aza-cytidine), seule la présence de $\beta$-mercapto-éthanol est nécessaire, mais pas suffisante.

La présente invention a donc pour objet un procédé d'obtention de cellules cardiaques, caractérisé en ce qu'il comprend au moins les étapes suivantes :

a) sélection de cellules cardiomyogéniques à partir de la fraction stroma-vasculaire (FSV ou SVF pour *stromal vascular fraction*) brute ;
b) culture des cellules sélectionnées à l'étape a) dans un milieu liquide optimisé pour l'expansion *ex vivo* des cellules cardiomyogéniques, lequel milieu liquide est sélectionné dans le groupe constitué par le milieu BHK21 contenant au moins du sérum de veau foetal et du $\beta$-mercapto-éthanol et tout autre milieu dont la composition est du même type que celle du milieu BHK21 en ce qui concerne la composition en sels inorganiques, acides aminés et vitamines ;
c) entretien et expansion desdites cellules par passages successifs en milieu liquide ; et
d) obtention de cellules cardiaques.

Ainsi, les cellules de la FSV représentent une population cellulaire hétérogène qui peut être utilisée directement (fraction FSV brute fraîchement préparée) ou après culture.

Les cellules de la FSV après culture primaire correspondent à une sous-population de cellules adhérentes (fraction des cellules adhérentes de la FSV, également dénommée ADSC pour *adipose-derived stromal cells*).

De manière avantageuse, ladite culture de cellules sélectionnées à l'étape a) est réalisée dans un milieu liquide tel que défini ci-dessus et sur une surface d'adhésion appropriée (type gélatine, protéines d'adhésion, protéines de la matrice extra-cellulaire). Par exemple, ladite culture est réalisée dans des boîtes recouvertes de gélatine et contenant un milieu BHK21. En effet, les propriétés d'adhésion des cellules cardiogéniques d'intérêt et le milieu de culture liquide utilisé pour l'expansion sont importants.

La culture primaire en méthylcellulose jusqu'à l'apparition de cellules allongées contractiles permet la sélection de cellules qui sont capables d'initier un programme de différenciation cardiaque.

Ces cellules sont ensemencées dans un milieu liquide BHK21. Une expansion cellulaire est obtenue en ensemençant tous les 2 jours dans un milieu BHK21 les cellules en suspension (pas de traitement protéolytique).

*In vitro,* on obtient à la fois des cellules cardiomyogéniques proprement dites (précurseurs capables de s'engager dans une voie de différenciation cardiaque) et des cellules de type cardiaque (cellules en cours de différenciation cardiaque) ; en effet, les cellules cardiomyogéniques que l'on obtient *in vitro* se différencient pour une grande part en cellules de type cardiaque, mais la différenciation *in vitro* n'est jamais aussi aboutie que celle obtenue *in vivo ; in vivo* l'on obtient des cardiomyocytes.

Lesdites cellules cardiaques peuvent avantageusement être conservées sous forme congelée.

Dans un premier mode de mise en oeuvre avantageux dudit procédé (procédé 1), la sélection de l'étape a) est effectuée par culture primaire de cellules de la fraction stroma-vasculaire (FSV ou SVF pour *stromal vascular fraction*) brute dans un milieu semi-solide, jusqu'à l'émergence de clusters (ou clones) de cellules contractiles ; dans ce cas :

- l'étape b) comprend le prélèvement desdites cellules contractiles constituées de deux sous populations présentant des types morphologiques distincts, à savoir des cellules adhérentes de type allongé et des cellules non-adhérentes de type rond (ou arrondi) ; et le repiquage desdites cellules contractiles dans un milieu liquide optimisé pour l'expansion *ex vivo* des cellules cardiomyogéniques ou des cellules souches embryonnaires (cellules de type ES) ; de préférence, l'étape b) de culture des cellules sélectionnées en a) est effectuée dans un milieu liquide BHK21 contenant au moins du sérum de veau foetal et du $\beta$-mercapto-éthanol et tout autre milieu dont la composition est du même type que celle du milieu BHK21 en ce qui concerne la composition en sels inorganiques, acides aminés et vitamines et sur une surface d'adhésion appropriée pour l'expansion *ex vivo* des cellules cardiomyogéniques.
- l'étape c) comprend l'entretien et l'expansion d'au moins l'une des deux sous populations de cellules (cellules adhérentes et cellules non adhérentes en suspension) par passages successifs en milieu liquide.
- le milieu semi solide est avantageusement sélectionné dans le groupe constitué par les dérivés cellulosiques (méthylcellulose, notamment) et les matrices membranaires basales reconstituées comprenant au moins l'un des éléments suivants : collagène, laminine et protéoglycanes (Matrigel, par exemple).

Les cellules adhérentes et les cellules non-adhérentes (ou en suspension dans le milieu liquide) présentent au moins les caractéristiques suivantes ; ces deux types cellulaires sont : CD44$^+$, CD81$^+$, CD 31$^-$, CD45$^-$, ckit$^-$ ; au moins 50% des deux populations sont positives pour Sca-1 et troponine T. Ces deux populations sont également CD90$^-$, Flkl$^-$ et MLC2v$^+$.

Ainsi, ces deux populations expriment CD44$^+$ et CD81$^+$ mais n'expriment pas CD31$^-$, CD34$^-$, CD45$^-$, CD90$^-$, CD117$^-$ (c-kit) et Flk1$^-$.

Ce phénotype antigénique est très particulier.

Ces deux populations sont également positives pour CD29 (cellules adhérentes : 98 % ; cellules non-adhérentes : 89 %) ; ce marqueur n'est toutefois pas spécifique de ces cellules.

La différence majeure entre les deux types cellulaires est la suivante : les cellules adhérentes sont positives pour CD38 et CD73 et quelques unes (20%) expriment CD34 et négatives pour CMH2 ; alors que les cellules non-adhérentes sont CMH1$^-$.

De manière avantageuse, la fraction stroma-vasculaire brute cultivée en milieu semi solide contenant en particulier de la méthylcellulose, suivi d'un repiquage des clusters contractiles en milieu liquide tel que le milieu BHK21, contenant au moins du sérum de veau foetal et du $\beta$-mercapto-éthanol, permet d'optimiser de manière significative, la transdifférenciation des cellules du tissu adipeux en cellules cardiomyogéniques dans la culture en milieu liquide. En effet, l'étape de culture primaire en milieu semi-solide, permet une sélection efficace des cellules cardiomyogéniques à partir de la fraction stroma-vasculaire et fournit, après ensemencement en milieu liquide (culture secondaire), un tapis cellulaire constitué des deux morphologies cellulaires cardiomyogéniques décrites (adhérente et en suspension). Cette culture peut être maintenue à long terme (plusieurs mois) par passages successifs (mécaniques ou enzymatiques), comparativement à un ensemencement direct dans le milieu liquide BHK21.

Dans ces conditions, on obtient, de manière surprenante de très bons rendements en cellules cardiomyogéniques :

- à partir des cellules en suspension : le tapis cellulaire adhérent est obtenu dans une boîte de culture de diamètre variable (30 mm de diamètre par exemple) et produit en moyenne 150 000 cellules cardiomyogéniques tous les deux jours ; ces dernières sont retrouvées en suspension dans le milieu de culture, et ceci pendant tout le temps de viabilité du tapis cellulaire, qui vieillit ensuite (~20 jours) et diminue sa production.
- à partir des cellules adhérentes : les cellules flottantes récupérées et placées dans une nouvelle boîte de culture, adhèrent et reforment un tapis adhérent en 12 jours. A partir de 150 000 cellules flottantes, 12 jours après on a un tapis constitué de 900 000 cellules adhérentes en moyenne.

Selon une disposition avantageuse de ce mode de mise en oeuvre (procédé 1), la durée de culture primaire selon l'étape a) est de quelques jours à quelques semaines, de préférence de une à deux semaines.

Selon une autre disposition avantageuse de ce mode de mise en oeuvre (procédé 1), préalablement au repiquage des cellules contractiles, une seconde sélection à l'aide d'au moins un marqueur approprié est effectuée ; ledit marqueur est avantageusement un marqueur positif (marqueur des cellules cardiaques) et/ou un marqueur négatif des cellules cardiaques.

Parmi les marqueurs positifs des cellules cardiaques, on peut citer : Sca-1, troponine, MLC2v, CD44, CD81, CD73, CD38.

Parmi les marqueurs négatifs des cellules cardiaques, on peut citer les marqueurs du complexe majeur d'histocompatibilité CMH (CMH1 ou CMH2)], CD31, CD34, CD45, c-kit et Flkl.

Le tri à l'aide d'un marqueur négatif et/ou positif, réalisé après culture sur milieu semi-solide, permet un enrichissement spectaculaire en cellules cardiomyogéniques. Par exemple, pour un ensemencement de 30.000 cellules CMH1 négatives, issues de la SVF brute par ml de méthylcellulose, on retrouve en moyenne 30 clusters de type cardiaque ; en conséquence, 1 cellule/1500 est à l'origine d'un cluster contractile après 14 jours de culture.

Selon une autre disposition avantageuse de ce mode de mise en oeuvre (procédé 1), le milieu liquide de l'étape b) est avantageusement un milieu BHK21 contenant au moins du sérum de veau foetal et du $\beta$-mercapto-éthanol ou tout autre milieu similaire susceptible d'être utilisé pour des cellules de type ES ; la composition dudit milieu est notamment du même type que celle du milieu BHK21 en ce qui concerne la composition en sels inorganiques, acides aminés et vitamines.

Dans un deuxième mode de mise en oeuvre avantageux dudit procédé (procédé 2), la sélection de l'étape a) est effectuée par tri cellulaire des cellules de la SVF brute à l'aide d'au moins un marqueur négatif des cellules cardiaques, tel que défini ci-dessus et donc notamment sélectionné dans le groupe constitué par les marqueurs du complexe majeur d'histocompatibilité (CMH1 ou CMH2) et CD31, CD34, CD45, c-kit et Flk1. En particulier, les cellules SVF capables de se différencier en cellules cardiomyogéniques sont de manière surprenantes CMH⁻.

Dans ce cas, l'étape b) comprend la culture des cellules sélectionnées dans un milieu liquide BHK21 contenant au moins du sérum de veau foetal et du $\beta$-mercapto-éthanol ou tout autre milieu similaire susceptible d'être utilisé pour des cellules de type ES, comme précisé ci-dessus.

Le tri effectué avec le marqueur négatif des cellules cardiaques et notamment le tri CMH est réalisé à partir d'une préparation fraîche de SVF. Le rendement de cellules CMH1 négatives obtenues après tri cellulaire est variable suivant les préparations de SVF et comprend, par exemple, 20 à 40 % de cellules CMH1⁻ à partir de SVF brute fraîchement préparée.

Quel que soit le mode de mise en oeuvre de l'étape a) (procédé 1 ou procédé 2), l'étape c) d'entretien et d'expansion des cellules cardiomyogéniques dans des cultures en milieu liquide peuvent être réalisés selon l'une des deux modalités suivantes :

- centrifugation des cellules en suspension dans un milieu liquide identique ou différent de celui de l'étape b) et réensemencement du culot cellulaire dans le même milieu liquide ; un nouveau tapis cellulaire se forme et à nouveau les deux types de morphologies cellulaires sont retrouvés. La culture des deux types de morphologies peut ainsi être maintenue à long terme ;

- décrochement enzymatique (type trypsine) ou tout autre procédé permettant le détachement des cellules du tapis cellulaire adhérent, centrifugation de la suspension cellulaire décrochée et réensemencement du culot cellulaire dans le même milieu liquide ; de façon similaire, un nouveau tapis cellulaire se forme et à nouveau les deux types de morphologies cellulaires sont retrouvés.

La culture des deux types de morphologies peut ainsi être maintenue à long terme.

De manière préférée, le procédé selon l'invention comprend :

a) une culture primaire de cellules de la FSV dans un milieu semi-solide à base de méthylcellulose et la sélection des cellules cardiomyogéniques,

b) une culture secondaire des cellules sélectionnées en a) dans un milieu liquide BHK21 contenant au moins du sérum de veau foetal et du $\beta$-mercapto-éthanol et tout autre milieu dont la composition est du même type que celle du milieu BHK21 en ce qui concerne la composition en sels inorganiques, acides aminés et vitamines et sur une surface d'adhésion appropriée pour l'expansion *ex vivo* des cellules cardiomyogéniques,

c) l'entretien et l'expansion desdites cellules par passages successifs en milieu liquide et

d) l'obtention de cellules cardiaques.

En variante, les étapes a) et b) sont fusionnées et peuvent être effectuées par culture primaire des cellules de la FSV en milieu BHK21 et sur un support ou surface d'adhésion approprié, tel que gélatine, protéines d'adhésion et protéines de la matrice extracellulaire.

Conformément à l'invention, la fraction stroma-vasculaire brute est obtenue, préalablement à l'étape a) à partir de dépôts de tissu adipeux par :

- isolement de la SVF par digestion par des enzymes protéolytiques et par séparation physique, notamment par combinaison d'étapes de centrifugation, et de filtration et/ou différence de densité et

- purification des cellules par séparation physique (filtration et/ou centrifugation) et/ou immunosélection.

Lesdites cellules cardiomyogéniques peuvent en outre être génétiquement modifiées. Ainsi :

- elles peuvent comprendre au moins une mutation d'un gène auto-logue ou
- elles peuvent contenir au moins une copie d'un gène hétérologue.

Lesdites cellules génétiquement modifiées sont, de préférence, d'origine humaine.

La présente invention a également pour objet l'utilisation des cellules cardiaques susceptibles d'être obtenues à partir des cellules de la fraction stroma-vasculaire, dans les conditions exposées dans les procédés tels que définis ci-dessus (procédé 1 ou procédé 2), pour la préparation d'un médicament apte à reconstituer une zone cardiaque ischémique.

Selon un mode de réalisation avantageux de ladite utilisation, lesdites cellules cardiaques sont sélectionnées dans le groupe constitué par :

(i) les cellules cardiomyogéniques contractiles adhérentes de forme allongée présentant les caractéristiques suivantes : $CD38^+$, $CD44^+$, $CD73^+$, $CD81^+$, $CD31^-$, $CD45^-$, $Ckit^-$, $CD90^-$, $Flk1^-$, $CMH2^-$, environ 50 % desdites cellules étant $Sca-1^+$, troponine $T^+$ et $MLC2v^+$,

(ii) les cellules non-adhérentes de forme arrondie : $CD44^+$, $CD81^+$, $CD31^-$, $CD45^-$, $CD73^-$, $CD90^-$, $Flk1^-$, environ 50 % desdites cellules étant $Sca-1^+$, troponine $T^+$, $MLC2v^+$ et $CMH1^-$,
les deux types de cellules exprimant en outre le facteur de transcription mésodermique Brachyury, les facteurs de transcription Islet-1 et MEF-2c et le facteur de liaison transcriptionnel Oct3/4, ou

(iii) un mélange desdites cellules adhérentes et desdites cellules non-adhérentes.

Ces deux types de cellules (adhérentes et non-adhérentes) sont également $CD34^-$.

Les inventeurs décrivent une composition pharmaceutique, caractérisée en ce qu'elle comprend un mélange :

(i) de cellules cardiomyogéniques adhérentes de forme allongée présentant au moins les caractéristiques suivantes : CD38+, $CD44^+$, $CD73^+$, $CD81^+$, $CD31^-$, $CD45^-$, $Ckit^-$, $CMH2^-$, environ 50 % desdites cellules étant $Sca-1^+$, troponine $T^+$ et $MLC2v^+$, et

(ii) de cellules non-adhérentes de forme arrondie présentant au moins les caractéristiques suivantes : $CD44^+$, $CD81^+$, $CD31^-$, $CD45^-$, $CD73^-$, $CMH1^-$, environ 50 % desdites cellules étant $Sca-1^+$, troponine $T^+$ et $MLC2v^+$,
les deux types de cellules exprimant en outre le facteur de transcription mésodermique Brachyury, les facteurs de transcription Islet-1 et MEF-2c et le facteur de liaison transcriptionnel Oct3/4, et au moins un véhicule pharmaceutiquement convenable.

Selon un mode de réalisation avantageux de ladite composition, elle comprend en outre un ou plusieurs facteurs cardiaques.

Selon une disposition avantageuse de ce mode de réalisation, ladite composition comprend en outre de la troponine T et/ou du MLC2v (Myosin Light Chain kinase 2v).

Ladite composition est administrée par n'importe quelle voie d'administration. En particulier, elle est avantageusement *injectée in situ* (injection directe) à l'aide de seringues appropriées ou par l'intermédiaire d'un cathéter endoventriculaire ou endocoronaire ; elle peut également être injectée par voie intra-veineuse ou administrée par voie systémique.

S'agissant de pathologies ischémiques, la masse tissulaire à reconstituer ou à implanter, s'avère considérable à l'échelle cellulaire (20 à 30 $cm^3$). Toutefois, dans la mesure où il s'agit de cellules à potentiel prolifératif (démontré *in vitro*), les quantités à injecter sont moindres que s'il s'était agi de cellules différenciées.

Ainsi, le procédé selon l'invention permet l'obtention de quantités importantes de cellules à potentiel cardiaque.

Or, l'infarctus du myocarde est associé à une perte importante de cardiomyocytes différenciés, en raison de l'ischémie. Cette perte ne peut pas être compensée par le renouvellement naturel des cardiomyocytes différenciés dans le coeur. Les cellules obtenues selon le procédé selon l'invention implantées dans un environnement ischémique aigu survivent et acquièrent effectivement les caractéristiques des cellules cardiaques avec des effets bénéfiques évitant le remodelage et la détérioration de la fonction ventriculaire gauche.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

- Figure 1 :

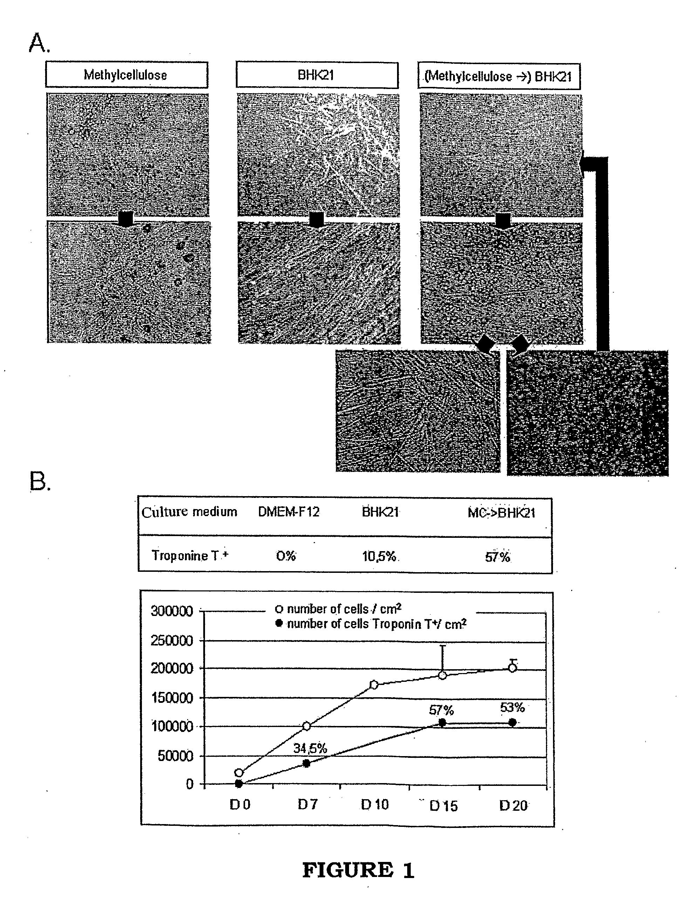

Figure 1A : évolution de la morphologie des cellules de la SVF cultivées dans différentes conditions :

- en méthylcellulose à un stade précoce (J7, haut) et plus avancé (J30, bas),

- en milieu liquide BHK21 à un stade précoce (J7, haut) et plus avancé (J15, bas),
- en méthylcellulose puis BHK21 après sélection à un stade précoce (J7, haut) et plus avancé (J15, milieu) donnant lieu à une population adhérente (bas, gauche) et une population en suspension (bas, droite) capable d'auto-entretenir la culture.

Figure 1B : pourcentage de cellules exprimant le marqueur cardiaque troponine T dans les différentes conditions de culture (haut) et évolution du nombre de cellules troponine T[+] dans les conditions de culture méthylcellulose (MC)→BHK21.

- Figure 2 : phénotypage (FACS) des deux populations cellulaires obtenues dans les conditions de culture MC→BHK21. (A-B) Représentation taille/granulosité et marquage troponine T. (C) Expression de différents marqueurs cellulaires.

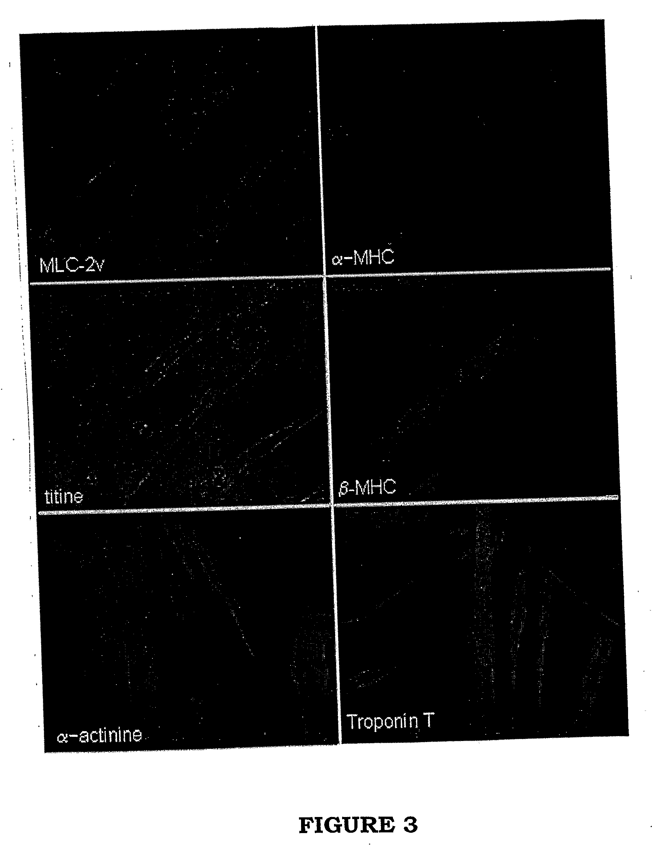

- Figure 3 : Différenciation cardiaque *in vitro* des cellules adhérentes : immunocytochimie montrant que les cellules expriment MLC-2v, titine, $\alpha$-actinine, $\beta$-MHC et troponine T. Les cellules n'expriment pas $\alpha$-MHC à ce stade.
- Figure 4 : Expression de gènes codant pour des facteurs de transcription de cellules souches (Oct3/4, Islet1), de cellules mésodermiques (Brachyury), de cellules cardiaques (Islet1, MEF2c).
- Figure 5 : Présence de cellules 7 jours après leur greffe dans un modèle murin d'ischémie du myocarde : immunohistochimie révélant la présence de rares (quand ce sont des cellules de la SVF cultivées en condition classique ou seulement les cellules adhérentes du tapis cellulaire) ou de nombreuses (adhérentes + suspension) cellules GFP dans des zones présentant un infarctus.
- Figure 6 : Identification par immunohistochimie de multiples sites où les cellules sont retrouvées 7 jours après leur greffe. (a) reconstitution d'une zone greffée avec de nombreuses cellules GFP. (b, c) marquage GFP spécifique de cellules retrouvées dans l'infarctus aussi bien que les zones bordantes comparativement au contrôle isotypique (b', c').

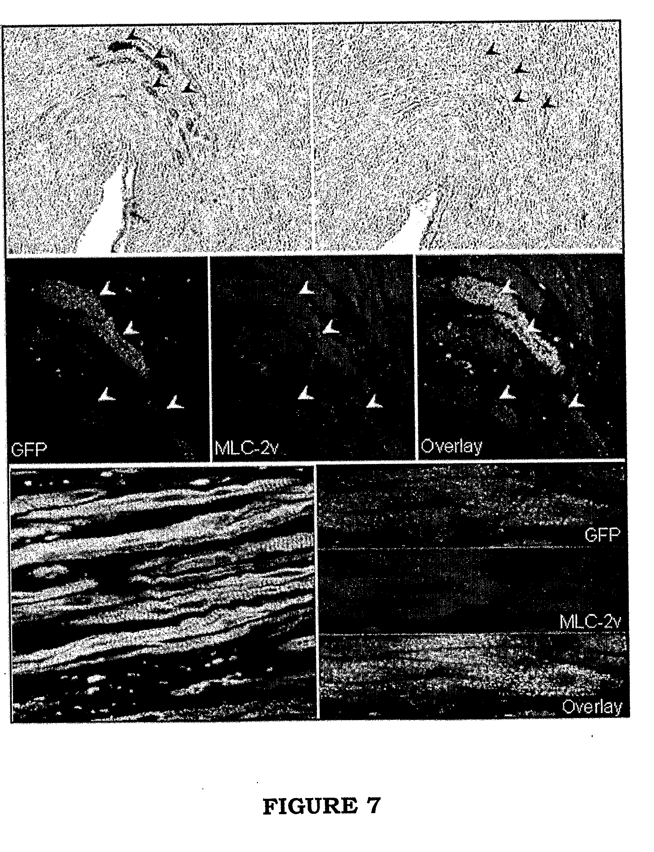

- Figure 7 : Différenciation cardiaque *in vivo* : immunohistochimie confocale montrant que les cellules greffées GFP[+] expriment aussi la MLC2v avec la co-expression visualisée dans l'image de superposition « overlay » , contrairement au tissu sain qui n'exprime que la protéine cardiaque (MLC2v) (voir flèches). Plus précisément : en haut : immunohistochimie pour la présence de GFP (flèches, figure de gauche) *versus* contrôle isotopique (figure de droite). Milieu et bas : immunofluorescence en microscopie confocale utilisant des anticorps anti-MLC2v et anti-GFP. La figure « overlay » montre la présence de myocytes cardiaques et de cellules co-exprimant les protéines GFP et MLC2v (gris clair).

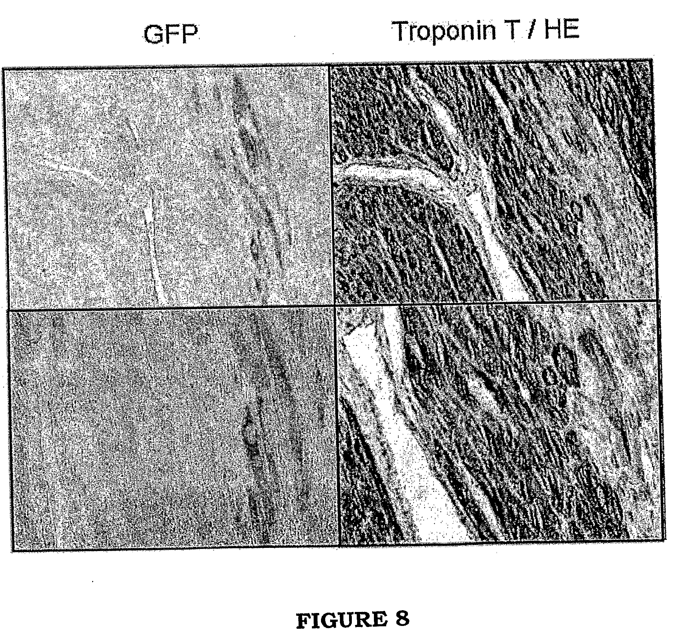

- Figure 8 : Différenciation cardiaque *in vivo* : immunohistochimie sur coupes sériées montrant que dans la même zone co-localisent des cellules exprimant la GFP (gauche) et le marqueur cardiaque troponine T (droite).
- Figure 9 : Fréquence d'apparition des clones cardiomyogéniques en méthylcellulose.
- Figure 10 : PCR quantitative pour déterminer la greffe des cellules *GFP in vivo.* Pourcentage de cellules adhérentes et non-adhérentes exprimant la GFP, implantées dans le coeur receveur du contrôle (animal sacrifié immédiatement après l'injection) et 7 jours après dans un modèle aigu d'infarctus du myocarde.
- Figure 11 : Analyse fonctionnelle par échocardiographie. A : évolution des volumes télésystoliques (LVESV, haut), volumes télédiastoliques (LVEDV, milieu) et fraction d'éjection du ventriculaire gauche (LVEF, bas) 0, 7 et 28 jours après injection des cellules ou du milieu. B : comparaison des variations des valeurs fonctionnelles entre J0 et J28.

Il doit être bien entendu, toutefois, que ces exemples sont donnés uniquement à titre d'illustration de l'objet de l'invention, dont ils ne constituent en aucune manière une limitation.

## EXEMPLE 1 : Matériels et Méthodes

### 1) Animaux et échantillons de tissus

Des souris C57B1/6 mâles âgées de 8 semaines (Harlan, France) et des souris mâles GFP sont élevées dans un environnement contrôlé (cycle lumière/obscurité de 12 h à 21°C) avec accès libres à l'eau et à la ration alimentaire standard.
Toutes les procédures sont réalisées en accord avec la Directive CEE/n° 07430.
A l'issue des expériences, les souris sont tuées par dislocation cervicale sous anesthésie au $CO_2$.
Le tissu adipeux est rapidement prélevé et traité pour les analyses ultérieures.

2) Isolement des cellules du tissu adipeux

Procédé 1 :

Les cellules sont isolées à partir du tissu adipeux de souris âgées de 5 à 8 semaines, globalement, selon le procédé décrit par Bjôntorp et al., 1978, avec des modifications mineures. Le tissu adipeux prélevé est disséqué sous microscope dans des boites stériles contenant du PBS, de manière à enlever toute trace de tissu musculaire, puis digéré à 37°C pendant 30 min, dans du milieu de digestion contenant du DMEM F12-OK, 2 % de BSA (albumine sérique bovine) et 2 mg/ml de collagénase (référence SIGMA) à raison de 10 ml de milieu de digestion pour 3 g de tissu. Le milieu DMEM F12-OK comprend pour 500 ml de DMEM F12 (référence Gibco 31330 038), 5 ml d'ASP (solution prête à l'emploi d'antibiotiques+antifongique : Amphotéricyne 0,25 $\mu$g/ml, Streptomycine 100 $\mu$g/ml, Pénicilline G 100 $\mu$g/ml) (référence SIGMA A7292), 0,016 mM de biotine (référence SIGMA B4639), 0,018 mM d'acide panthoténique (référence SIGMA P5155) et 100 $\mu$M d'acide ascorbique (référence SIGMA A4034). Après élimination des fragments non digérés par filtration (filtres de 25 $\mu$m), les adipocytes matures sont séparés du culot contenant les cellules de la SVF par centrifugation (600 g, 10 min). Les cellules stroma-vasculaires ainsi isolées sont remises en suspension dans du milieu de culture DMEM-F12 et comptées (comptage manuel sur cellule hématimétrique ou compteur à particules Coulter).

Procédé 2 :

Les cellules sont isolées à partir du tissu adipeux de souris âgées de 3 semaines et on procède comme décrit dans le procédé 1 en mettant en oeuvre 10 ml de milieu de digestion pour 1 g de tissu adipeux. Les conditions de digestion sont identiques à celles du procédé 1, le milieu de digestion est différent. Il contient 6,7 mM Hepes, 3 mM NaCl, 0,025 mM KCl, 0,078 mM CaCl$_2$, 4,5 mg/ml glucose, 1,5 % BSA additionné de 1 mg/ml de collagénase. En variante, le milieu de digestion est un tampon PBS contenant 2 % de BSA et 2 mg/ml de collagénase (utilisé notamment pour le tissu adipeux brun interscapulaire extrait de souris transgénique GFP). Après élimination des fragments non digérés par filtration (filtres de 25 $\mu$m), les adipocytes matures sont séparés du culot contenant les cellules de la SVF par centrifugation (1 000 g, 10 min).
Les cellules stroma-vasculaires ainsi isolées sont remises en suspension dans du milieu de culture DMEM-F12 et une nouvelle centrifugation est réalisée (1000 g, 10 min). Les cellules sont remises en suspension dans du milieu de culture et comptées comme précédemment.

3) Culture sur milieu à base de méthylcellulose

Les cellules SVF isolées (fraction SVF brute fraîchement préparée) comme précisé en 2), sont mises en culture (30 000 cellules/ml) dans un milieu à base de méthylcellulose (Methocult GFM3534, StemCell Technologies, Vancouver) et maintenues en culture pendant deux semaines, pendant lesquelles la morphologie des clones (ou clusters) obtenus est suivie.
Les clones contractiles sont repérés, prélevés et lavés dans du PBS.
Après une centrifugation rapide (5 min, 600 g), une suspension cellulaire est préparée pour une utilisation ultérieure.

4) Procédure de culture des clones contractiles

Un extrait brut de cellules SVF (32 000 cellules/cm$^2$) ou des clones prélevés à partir du milieu à base de méthylcellulose (1 500 cellules/cm$^2$) sont ensemencés dans des boîtes de 30 mm (Greiner Bio-One) recouvertes de gélatine à 0,1 % et mis en culture dans un milieu BHK21 (Gibco BRL), -dont la composition précise est fournie dans les Tableaux I et II ci-après-, contenant 10 % de sérum de veau foetal (StemCell Technologies) et supplémenté avec 10$^4$M de $\beta$-mercapto-éthanol (Sigma), 2 mM de glutamine (Gibco BRL), 1 mM de pyruvate (Gibco BRL), 0,1 mM d'acides aminés non essentiels (Gibco BRL) et une solution contenant 0,25 $\mu$g/ml d'amphotéricine, 100 U/ml de pénicilline G et 100 $\mu$g/ml de streptomycine (Sigma, solution APS).
Tout autre milieu similaire au milieu BHK21, susceptible d'être utilisé pour les cellules de type ES peut également être mis en oeuvre. La composition dudit milieu est notamment du même type que celle du milieu BHK21 en ce qui concerne la composition en sels inorganiques, acides aminés et vitamines.
L'expansion cellulaire est réalisée par récolte des cellules en suspension dans le milieu de culture tous les trois jours. Après centrifugation (600 g, 5 min), les cellules sont réensemencées dans des boîtes de culture de 30 mm de diamètre recouvertes de gélatine à 0,1 % et mises en culture dans un milieu BHK21.

5) Culture de cellules contrôles

Les cellules SVF brutes fraîchement préparées sont ensemencées dans du milieu DMEM-F12, dont la composition précise est fournie dans le Tableau III, supplémenté par 10 % de sérum de veau nouveau-né, à une densité de 30 000 cellules/cm$^2$. Ces cellules sont dénommées cellules ADSC (*Adipose-Derived Stromal Cells*).

Pour les expériences contrôles, ces cellules sont en outre traitées comme suit : Après 6 heures de culture, les cellules non-adhérentes sont éliminées par lavage et les cellules adhérentes sont maintenues en culture pendant six jours jusqu'à phénotypage ou greffe (pour expansion). Les cellules sub-confluentes sont récoltées par trypsinisation 5 min à 37°C et constituent la fraction des cellules adhérentes de la FSV. Ces cellules sont transplantées dans les souris.

TABLEAU I

| Composition du milieu de culture liquide BHK21 | |
|---|---|
| Réf. Composants | 21710 (ou tout milieu susceptible d'être utilisé pour les cellules de type ES) 1X liquide mg/L |
| **SELS INORGANIQUES :** | |
| $CaCl_2$ o $2H_2O$ | 265,00 |
| $Fe(NO_3)_3$ o $9H_2O$ | 0,10 |
| KCl | 400,00 |
| $MgSO_4$ o $7H_2O$ | 200,00 |
| NaCl | 6400,00 |
| $NaHCO_3$ | 2750,00 |
| $NaH_2PO_4$ o $2H_2O$ | 141,00 |
| | |
| **ACIDES AMINÉS :** | |
| L-arginine o HCl | 42,00 |
| L-cystine | 24,00 |
| L-glutamine | 292,00 |
| L-histidine HCl o $H_2O$ | 21,00 |
| L-isoleucine | 52,00 |
| L-leucine | 52,00 |
| L-lysine o HCl | 73,00 |
| L-méthionine | 15,00 |
| L-phénylalanine | 33,00 |
| L-thréonine | 47,60 |
| L-tryptophane | 8,00 |
| L-tyrosine | 36,20 |
| L-valine | 46,80 |
| | |
| **ACIDES AMINES NON ESSENTIELS** (voir Tableau II) | 5 ml, 10 mM/500 ml |
| | |
| **VITAMINES :** | |
| Pantothénate de calcium D | 2,00 |
| Chlorure de choline | 2,00 |
| Acide folique | 2,00 |
| i-inositol | 3,60 |
| Nicotinamide | 2,00 |
| Pyridoxal HCl | 2,00 |
| Riboflavine | 0,20 |
| Thiamine HCl | 2,00 |
| | |
| **AUTRES COMPOSANTS :** | |

(suite)

| Composition du milieu de culture liquide BHK21 | |
|---|---|
| Réf. Composants | 21710 (ou tout milieu susceptible d'être utilisé pour les cellules de type ES) 1X liquide mg/L |
| D-Glucose | 4500,00 |
| Rouge de phénol | 15,00 |
| L-glutamine | 5 ml, 200 mM/500 ml |
| β-mercaptoéthanol | $10^{-4}$ M final |
| Pyruvate | 5 ml, 100 mM/500 ml |
| Antibiotiques-antimycosiques (amphotéricine, streptomycine, pénicilline) (ASP, Sigma A7292) | 1% |
| Sérum ES 10% (cellules souches) | 10 %1 |

TABLEAU II

| Acides aminés non essentiels | |
|---|---|
| Réf. Composants | 11140 100X liquide g/L |
| **ACIDES AMINÉS** | |
| L-alanine | 890,00 |
| L-asparagine | 1320,00 |
| L-acide aspartique | 1330,00 |
| L-acide glutamique | 1470,00 |
| Glycine | 750,00 |
| L-proline | 1150,00 |
| L-sérine | 1050,00 |

TABLEAU III

| Composition du milieu de culture liquide DMEM-F12 | |
|---|---|
| **Composés** | **Concentration (mg/L)** |
| **ACIDES AMINÉS :** | |
| Glycine | 18,75 |
| L-alanine | 4,45 |
| Hydrochlorure de L-arginine | 147,5 |
| L-Asparagine-$H_2O$ | 7,5 |
| Acide L-aspartique | 6,65 |
| Hydrochlorure $H_2O$ de L-cystéine | 17,56 |
| L-cystine 2HCl | 31,29 |
| Acide L-glutamique | 7,35 |
| Hydrochlorure $H_2O$ de L-histidine | 31,48 |
| L-isoleucine | 54,47 |
| L-leucine | 59,05 |
| Hydrochlorure de L-lysine | 91,25 |
| L-méthionine | 17,24 |
| L-phénylalanine | 35,48 |
| L-proline | 17,25 |
| L-sérine | 26,25 |
| L-thréonine | 53,45 |

(suite)

| Composition du milieu de culture liquide DMEM-F12 | |
|---|---|
| **Composés** | **Concentration (mg/L)** |
| L-tryptophane | 9,02 |
| Sel déhydraté disodique de L-tyrosine | 55,79 |
| L-valine | 52,85 |
| | |
| **VITAMINES :** | |
| Phosphate d'acide ascorbique | 2,5 |
| Biotine | 0,0035 |
| Chlorure de choline | 8,98 |
| Pantothénate de calcium D | 2,24 |
| Acide folique | 2,65 |
| i-inositol | 12,6 |
| Niacinamide | 2,02 |
| Hydrochlorure de pyridoxine | 2 |
| Riboflavine | 0,219 |
| Hydrochlorure de thiamine | 2,17 |
| Vitamine B12 | 0,68 |
| | |
| **SELS INORGANIQUES :** | |
| Chlorure de calcium ($CaCl_2$) (anhyd.) | 116,6 |
| Sulfate de cuivre ($CuSO_4$-$5H_2O$) | 0,0013 |
| Nitrate ferrique ($Fe(NO_3)_3$"$9H_2O$) | 0,05 |
| Sulfate ferrique ($FeSO_4$-$7H_2O$) | 0,417 |
| Chlorure de magnésium (anhyd.) | 28,64 |
| | |
| **AUTRES COMPOSANTS** | |
| Biotine (Sigma B4639) | 16 $\mu$M |
| Acide ascorbique (Sigma A4034) | 100 $\mu$M |
| Acide panthoténique (Sigma P5155) | 18 $\mu$M |
| Antibiotiques-antimycosiques (amphotéricine-streptomycine-pénicilline) (Sigma A7292) Sérum de veau nouveau-né | 1 % |
| | 10% |

6) Caractérisation phénotypique

Les cellules sont marquées dans du tampon PBS contenant 0,2 % de sérum de veau foetal et incubées avec des anticorps monoclonaux anti-souris conjugués à de l'isothiocyanate de fluorescéine (FITC), de la phycoérythrine (PE), de la protéine de chlorophylle péridinine (PerCP) ou de l'allophycocyanine (APC) pendant 30 min à 4°C.

Après lavages, les cellules sont analysées en cytométrie de flux (FACS ou *Fluorescence Analyse Cell Sorter*) (FACS Calibur, Becton Dickinson, Mountain View, California). L'acquisition et l'analyse des données sont réalisées à l'aide du logiciel Cell Quest (Becton Dickinson).

Tous les anticorps (CD34, CD31, CD38, CD44, CD45, CD73, CD81, CD90, CD117, Sca1 et Flk1) proviennent de BD Biosciences (Heidelberg, Allemagne).

Pour le marquage intracellulaire, après une fixation au paraformaldéhyde à 4 % pendant 10 min à 4°C, les cellules sont perméabilisées par du tampon PBS contenant 1 % de BSA et 0,5 % de saponine (Sigma) pendant 20 min à température ambiante.

Puis, après marquage avec des anticorps anti-troponine T (Microm Microtech, clone 13-11), un anticorps PerCP anti-souris est utilisé (BD Biosciences).

7) Immunocytochimie

Les cellules adhérentes, cultivées sur lames, sont lavées avec du PBS, puis fixées pendant une nuit à 4°C dans un mélange paraformaldéhyde à 3,7 %/tampon PBS, ou refroidies à -20°C dans un mélange méthanol-acétone (50/50).

Après blocage des sites non-spécifiques pendant 1 heure dans du tampon PBS contenant 1 % de BSA, les cellules sont incubées pendant 1 heure dans un mélange Triton X100 à 0,3 %/tampon PBS avec un anticorps primaire : anticorps de souris dirigés contre MLC2v (1:2, Biocytex, France), contre α-actinine sarcomérique (1:500, Sigma), contre anti-β-MHC (1:X), contre anti-α-MHC, contre la titine (1:X), ou contre la troponine T (1:100, Microm Microtech) ou des anticorps de lapin anti-connexine 43 (1:X, Zymed Laboratories).

Après lavage, un anticorps secondaire est ajouté et l'incubation est de 60 min à température ambiante : Alexa 546 ou Alexa 350 conjugué à des IgG anti-souris ou anti-lapin (Molecular Probes Eugene, OR, dilution 1:300).

Les contrôles négatifs (pas de coloration) sont réalisés avec des IgG de souris purifiées (Dako X0931) ou des IgG de lapin purifiées (Dako X0903) et n'entraînent aucune coloration.

8) Extraction de l'ARN et analyse PCR quantitative en temps réel

De préférence, la quantification est réalisée en temps réel, c'est-à-dire que la détection et la quantification du signal émis par la sonde, notamment l'émission de fluorescence, sont effectuées pendant le processus d'amplification, dans la mesure où l'augmentation du signal est directement proportionnelle à la quantité d'amplimères produits durant la réaction.

Les principes généraux de la PCR et de la RT-PCR quantitatives en temps réel, ainsi que les différentes techniques de détection quantitative des amplimères à l'aide de sondes fluorescentes, à savoir : hydrolyse de sondes par l'activité 5' nucléase de l'ADN polymérase (TaqMan™), hybridation de 2 sondes (Hybprobes), balises moléculaires (Molecular Beacons) et amorces scorpion (Scorpion primers) sont connues de l'Homme du métier et sont notamment décrites dans Poitras et al., Reviews in Biology and Biotechnology, 2002, 2, 1-11. La PCR et la RT-PCR quantitative en temps réel à l'aide de sondes du type TaqMan™ sont notamment décrites, respectivement dans Heid C. et al. (Genome Research, 1996, 6, 986-994) et Gibson U. et al. (Genome Research, 1996, 6, 995-1001).

Chacune des sondes est marquée, à au moins une extrémité, par un fluorochrome différent. De préférence, l'extrémité 5' est marquée par un fluorochrome émetteur (*reporter*) et l'extrémité 3' par un fluorochrome suppresseur (*quencher*).

Avantageusement, l'une des extrémités, de préférence l'extrémité 3' de chacune des sondes est également marquée par un groupement MGB (Minor Groove Binder), qui permet d'augmenter artificiellement la température d'hybridation de chacune des sondes et de réduire ainsi leur longueur.

Les fluorochromes émetteurs utiles pour la détection quantitative, sont connus de l'Homme du métier, il s'agit notamment de : 6-FAM (6-carboxy-fluorescéine), TET (tétrachloro-6-carboxyfluorescéine), HEX (hexachloro-6-fluorescéine), isothyocyanate de fluorescéine (FITC), rhodamine, cyanine (CY3, CY5) et rouge Texas (*Texas Red*).

De même, les fluorochromes suppresseurs sont connus de l'Homme du métier ; il s'agit notamment du rouge de méthyle et du TAMRA (6-carboxytétraméthylrhodamine).

Plus précisément, l'ARN total des cellules repérées comme contractiles en culture et l'ARN de coeur de souris sont isolés à l'aide du kit d'extraction de l'ARN (RNeasy, Quiagen, France).

Un μg d'ARN est transcrit en ADN à l'aide de la transcriptase inverse M-MLV (Invitrogen, Cergy, France) et est utilisé dans la PCR en temps réel.

Les séquences des amorces PCR sont les suivantes :

MEF2C

sens: 5'-AGATACCCACAACACACCACGCGCC-3' (SEQ ID NO:1)
antisens : 5'-ATCCTTCAGAGAGTCGCATGCGCTT-3' (SEQ ID NO:2)

Oct-3/4

sens: 5'-TCAGCTTGGGCTAGAGAAGG-3' (SEQ ID NO:3) antisens : 5'-TGACGGGAACAGAGGGAAAG-3' (SEQ ID NO:4) Brachyury
sens : 5'-GACTTCGTGACGGCTGACAA-3' (SEQ ID NO:5) antisens : 5'-CGAGTCTGGGTGGATGTAG-3' (SEQ ID NO:6) Islet1
sens : 5'-CATCGAGTGTTTCCGCTGTGTAG-3' (SEQ ID NO:7)
antisens : 5'-GTGGTCTTCTCCGGCTGCTTGTGG-3' (SEQ ID NO:8)

La PCR en temps réel est réalisée à l'aide d'un thermocycleur LightCycler rapide (Roche, Meylan, France). L'amplification est réalisée selon les recommandations du fabricant.

12 μl d'un mélange réactionnel contenant 10 μl d'un mélange LightCycler-DNA Master SYBR green I (FAST Start Kit comprenant *Taq*ADN polymérase, tampon de réaction, mélange de désoxynucléosides et colorant SYBR Green) ajouté à 3 mM MgCl$_2$, 0,5 μM d'un mélange d'amorces appropriées et 2 μl d'ADNc.

Les résultats sont exprimés en fonction du taux d'expression du gène d'intérêt, en utilisant un modèle mathématique antérieurement décrit (Pfaffl et al., Nucleic Acids Res., 2002, 30, 9, e36).

Les données sont normalisées par analyse PCR d'α-tubuline (étalon interne).

Le protocole d'amplification comprend une dénaturation initiale à 95°C pendant 8 min, suivie de 40 cycles comprenant une étape de dénaturation à 95°C pendant 3 s, une étape d'hybridation à 65°C pendant 10 s et une étape d'élongation à 72°C pendant 10 s.

Le taux de transition de température est de 20°C/s.

La fluorescence est mesurée à la fin de chaque étape d'élongation.

Après amplification, une courbe de fusion est réalisée par chauffage du produit à 20°C/s jusqu'à 95°C, refroidissement à 20°C/s jusqu'à 70°C, et maintien à 70°C pendant 20 s puis en chauffant lentement à 0,1 °C/s jusqu'à 95°C.

La fluorescence est mesurée pendant la phase de chauffage lent.

Les courbes de fusion sont utilisées pour déterminer la spécificité des produits de PCR, ce qui est confirmé par électrophorèse sur gel.

Le test *t* de Student est utilisé pour analyser la significativité statistique. Toutes les valeurs P correspondent à des tests effectués en double et des P<0,05 sont considérés comme statistiquement significatifs.

L'analyse PCR peut également être réalisée à partir de l'ADN.

L'ADN de coeur de souris est isolé à l'aide du kit d'extraction QIAmp® DNA mini kit (QIAGEN).

Les concentrations en ADN sont mesurées avec le système Nanodrop ND100.

La pureté de l'ADN est vérifiée par des mesures d'absorbance à 260 et 280 nm.

La PCR en temps réel est réalisée avec le thermocycleur AB17000 (Applied Biosystem) pour quantifier les cellules greffées dans le coeur de souris. Chaque milieu réactionnel (25 μl) contient entre 1 et 3 ng d'ADN génomique, 0,3 μM de chaque amorce et 2,5 μl d'un mélange 2x SYBR Green master mix (Applied Biosystem).

Les données sont normalisées par analyse PCR du gène 36B4 (phosphoprotéine PO ribosomale acide). Les séquences nucléotidiques des amorces PCR sont les suivantes :

- GFP

    sens : GGGCACAAGCTGGAGTACAAC (SEQ ID NO:9)
    antisens : TCACCTTGATGCCGTTCTTCT (SEQ ID NO:10)

- 36B4

    sens : AGTCGGAGGAATCAGATGAGGAT (SEQ ID NO:11).
    antisens : GGCTGACTTGGTTGCTTTGG (SEQ ID NO:12)

Le protocole d'amplification comprend une dénaturation initiale à 95°C pendant 10 min, suivie de 40 cycles comprenant une étape de dénaturation à 95°C pendant 15 sec et une étape d'élongation à 60°C pendant 1 min. Pour confirmer l'absence d'amplification non spécifique, une courbe de dissociation est générée de 60°C à 95°C.

La courbe standard pour quantifier les gènes GFP et 36B4 dans le coeur de la souris greffée est réalisée en mélangeant l'ADN GFP de la souris donneuse avec l'ADN C57B1/6N de la souris greffée.

9) <u>Modèle d'infarctus du myocarde</u>

Des souris femelles C57 B1/6N âgées de 8 à 12 semaines (22 à 28 g) sont utilisées. Comme elles sont très proches génétiquement des souris donneurs GFP [souris transgéniques exprimant la GFP (*Green Fluorescent Protein*)] (Okabe M. et al., FEBS Lett., 1997, 407, 3, 313-319), un traitement immunosuppresseur n'est pas nécessaire.

Après une injection intrapéritonéale de kétamine (75 mg/kg) et de xylasine (7,5 mg/kg), les souris sont soumises à une intubation trachéale pour permettre une ventilation (ventilateur Hugo Sachs elektronik, March-Hugstetten, Allemagne).

Une anesthésie gazeuse est maintenue avec de l'isoflurane ou de l'halotane à 2 %.

L'intervention chirurgicale est effectuée sous agrandissement optique (4 et 8 X).

Après thoracotomie gauche jusqu'au 5ème espace intercostal, le péricarde est ouvert et l'artère descendante gauche est ligaturée avec un fil en polypropylène 8/0 (Ethicon, Johnson & Johnson, Bruxelles). Immédiatement après, un volume total de 10 μl contenant $1.10^5$ cellules est injecté en 3 points dans la zone ischémique (zone pâle, en aval de la ligature) avec une seringue Hamilton n° 701 (Reno, Nevada).

Le thorax est refermé et les souris sont réveillées.

Pour les études fonctionnelles échocardiographiques, le modèle d'infarctus du myocarde aigu est légèrement modifié.

Pour obtenir des données avant (point de comparaison) et 3 jours après l'infarctus du myocarde, un modèle sub-aigu est utilisé.

Les injections des cellules ou du milieu (groupe contrôle) sont réalisées 3 jours après la ligature de l'artère coronaire, à l'aide de la même approche intercostale. Une échographie contrôle est effectuée avant génération de l'infarctus et donne la valeur basale (baseline). Une échographie est ensuite réalisée 3 jours après l'infarctus et correspond à J0. L'injection de cellules selon l'invention ou de milieu est alors réalisée.

Tous les types cellulaires testés sont injectés en même quantité ($10^5$) et remis en suspension dans le même volume (10 μl).

10) Immunohistochimie

Les souris sont tuées par dislocation cervicale sous anesthésie au $CO_2$. Les coeurs sont rapidement prélevés, les 7e, 14e et 28e jours, coupés longitudinalement selon l'axe central et fixés une nuit à 4°C dans du paraformaldéhyde à 3,7 % ou à 4 %. Le tissu est inclus dans de la paraffine et coupé en sections de 6 μm.

Pour l'immunohistologie, les sections de tissu sont déparaffinées pendant 15 minutes et réhydratées dans de l'alcool. L'activité peroxydasique endogène est inhibée par incubation dans du peroxyde d'hydrogène à 3 % pendant 20 minutes puis 2 lavages de 5 minutes dans du PBS. Ces sections sont ensuite incubées pendant 30 minutes dans du BSA à 1 % dans du PBS, puis avec un anticorps primaire : anticorps de lapin anti-GFP (1:300, Molecular Probe A11122) et/ou anticorps de souris anti-MLC-2v (1:2) ou anticorps de souris anti-troponine T (1:100). En ce qui concerne ce dernier anticorps, une incubation dans un tampon citrate à 95°C pendant 25 min est nécessaire avant le marquage. Après lavages, les anticorps secondaires suivants sont utilisés : anticorps FITC de chèvre anti-lapin (1:500, Jackson) anticorps Rouge Texas d'âne anti-souris (1:100, Jackson), anticorps HRP d'âne anti-lapin (1:500, Jackson) ou anticorps HRP d'âne anti-souris (1:500, Jackson). Pour l'anticorps HRP secondaire, le marquage sur les coupes est révélé par l'AEC (Dako) et visualisé sous microscope inverse (Leica, TMRB), l'analyse de la fluorescence est réalisée avec un microscope confocal (Zeiss, LSM 510).

11) Evaluation fonctionnelle par échocardiographie

L'évaluation fonctionnelle échocardiographique est réalisée dans les deux groupes, avant l'infarctus du myocarde (point de comparaison), immédiatement avant l'injection (J0, 3 jours après l'infarctus du myocarde), 1 semaine (J7) et 4 semaines (J28) après l'injection.

Toutes les analyses échocardiographiques sont effectuées sous anesthésie gazeuse. Les évaluations échographiques sont réalisées avec le système échocardiographique VIVID 7 (General Electric) équipé d'un transducteur à matrice linéaire 14 MHz (Agilent; Andover MA), ce qui permet d'obtenir une coupe transverse sur le ventricule gauche. Les télésystoles et les télédiastoles sont définies comme les phases dans lesquelles la surface du ventricule gauche est la plus petite ou la plus grande, respectivement.

Le diamètre du ventricule gauche en télésystole (*Left-hentricular $E_h$d Systolic Diamenter* ou LVESD) et le diamètre du ventricule gauche en télédiastole (*Left Ventricular End-Diastolic Diameter* ou LVEDD) sont mesurés au niveau du muscle papillaire dans le M-mode de traçage du ventricule gauche avec une vitesse de balayage de 200 mm/s.

D'autres paramètres cardiaques sont également mesurés en 2-D.

La fraction d'éjection (*Ejection Fraction* ou EF) est mesurée par la surface dans un seul plan et calculée selon la formule suivante :

$$\text{EF (\%)} = [(0.85 \ \text{LVAD}^2/\text{LVLD}) - (0.85 \ \text{LVAS}^2/\text{LVLS})]/(0.85 \ \text{LVAD}^2/\text{LVLD}) \times 100,$$

dans laquelle LVAD correspond à la surface télédiastolique du ventricule gauche (*Left Ventricular Area in end-Diastole*) ; LVAS correspond à la surface télésystolique du ventricule gauche ; LVLD correspond à l'épaisseur du ventricule gauche en télédiastole selon un axe longitudinal (*Left Ventricular Long Axis leugth in end-Diastole*) et LVLS correspond à l'épaisseur du ventricule gauche en télésystole selon un axe longitudinal.

Chaque valeur est la moyenne de trois mesures.

Toutes les données sont exprimées en moyennes ± SEM.

Le test *t* de Student est utilisé pour analyser la significativité statistique. Toutes les valeurs P correspondent à des tests *t* appariés effectués pour chaque groupe suivi et à des tests *t* non appariés pour la comparaison des groupes.

Tous les P<0,05 sont considérés comme statistiquement significatifs.

## EXEMPLE 2 : Différenciation des cellules de SVF en cellules de type cardiaque

### 2.1. : Milieux standards

Le milieu liquide standard DMEM-F12 contenant 10 % de sérum de veau nouveau-né, ou le milieu BHK21 contenant 10 % de sérum de veau foetal, classiquement utilisés pour la culture des cellules souches pluripotentes, ont été testés en comparaison avec la méthylcellulose (contrôle positif ; figure 1A).

Quels que soient les éléments ajoutés (IL3, IL6, SCF, BMP2, TGF-$\beta$ en plus ou non d'une exposition à la 5-aza-cytidine), le milieu DMEM-F12 ne permet jamais d'obtenir une différenciation des cellules SVF en cellules cardiaques de manière efficace.

### 2.2. Conditions selon l'invention

### 2.2.1

Lorsque des cellules SVF brutes sont ensemencées et mises en culture dans un milieu BHK21 contenant 10 % de sérum de veau foetal et sur un support ou surface d'adhésion approprié (gélatine), environ 10% des cellules présentent un phénotype cardiaque estimé par contraction spontanée des cellules et expression de troponine T (figure 1B). L'élimination du $\beta$-mercaptoéthanol conduit à l'absence d'émergence de clusters de cellules cardiaques, mais l'addition de ce composé à d'autres milieux de culture classiques n'induit jamais une différenciation cardiaque, ce qui suggère que le $\beta$-mercaptoéthanol est nécessaire mais pas suffisant pour engager les cellules SVF dans une différenciation cardiaque, le milieu BHK21 étant également important.

La fréquence de l'obtention de clusters de phénotype cardiaque dans ces conditions de culture est variable, mais permet néanmoins d'obtenir suffisamment de cellules cardiaques en vue de leur transplantation.

### 2.2.2.

- culture primaire dans un milieu à base de méthylcellulose (voir exemple 1.3) jusqu'à l'émergence de clones contractiles (environ 2 semaines)
- sélection des cellules contractiles par dissection sous microscope inverse (sélection à la fois des cellules allongées et des cellules arrondies)
- culture des clones sélectionnés dans un milieu liquide BHK21, contenant au moins du sérum de veau foetal et du $\beta$-mercapto-éthanol, dans les conditions exposées à l'exemple 1.4), i.e. par ensemencement dans des boîtes recouvertes de gélatine et addition du milieu BHK21.

Dans les 48 heures qui suivent, après mise en culture dans le milieu BHK 21 liquide, on observe quelques fibres allongées et contractiles.

Deux semaines après, environ 60 % des cellules sont Troponine T$^+$ (figure 1B).

A cette étape, la culture contient deux types morphologiques distincts de cellules : des fibres contractiles qui adhèrent et des cellules arrondies attachées auxdites fibres, mais principalement présentes en suspension non-adhérentes.

Les cellules arrondies non-adhérentes sont peu attachées et peuvent être récupérées par collecte et centrifugation du milieu de culture.

Les cellules arrondies peuvent être à nouveau ensemencées dans du BHK21 liquide ; on obtiendra à nouveau, au bout de quelques jours les deux types de morphologies (fibres et cellules arrondies). Un examen attentif de la culture dérivée des cellules arrondies re-ensemencées sur milieu BHK21 liquide montre une expression élevée en troponine T (60-70 %) corrélée à une faible expression en CD90 (3-9%).

Par contre, les cellules exprimant fortement CD90 (60 %) présenteront un potentiel cardiaque faible en culture (30 % de troponine T$^+$ ou moins). Cette observation est corrélée avec la présence d'une sous-population de petites cellules fibroblastiques se développant dans le milieu de culture à la place des cellules allongées en forme de fibres et contractiles.

La culture à partir des cellules arrondies en suspension a été obtenue après plus de 10 passages (n = 3) ;

toutefois, les cellules sélectionnées pour une caractérisation ou une greffe chez la souris sont de préférence obtenues après 1 à 4 passages.

Une culture à long-terme des cellules arrondies (plus de 15 passages, n = 3) montre que ces cellules ont une morphologie stable.

La population de cellules adhérentes peut être récoltée par un traitement à la trypsine après lavage méticuleux pour éliminer la plupart des cellules arrondies, qui restent en suspension.

Pour éviter l'utilisation de la trypsine, il est donc possible pour obtenir une bonne expansion des cellules de récupérer et de réensemencer les cellules en suspension dans le milieu de culture (cellules arrondies).

Avec une telle procédure, $2.10^8$ cellules adhérentes Troponine T positives et $1,5.10^7$ cellules non-adhérentes peuvent être obtenues en 4 semaines, à partir d'un gramme de tissu graisseux ($20 \times 10^6$ cellules SVF brutes) sans utiliser de trypsine. Ceci permet une expansion extraordinaire. Ensemencées à 200 000 cellules par boîte de 30 mm (200.000/P30), ces cellules non adhérentes arrondies deviennent une couche confluente de $10^6$ cellules avec 60 % de cellules Troponine T positives en huit jours.

Simultanément, chaque plaque produit 150.000 cellules arrondies en suspension tous les deux jours pendant 10-15 jours pendant 4-6 semaines, c'est-à-dire au moins jusqu'à ce que la couche adhérente vieillisse.

2.3. Etudes comparatives

- Culture des cellules de la FSV dans du milieu DMEM-F12 uniquement :

  On obtient pas ou très peu de cellules cardiogéniques. Les cellules sont perdues au premier changement de milieux (15 min à 4h après ensemencement) car elles n'adhèrent pas ou très peu au flacon de culture dans ces conditions. Si on récolte le milieu de culture contenant les cellules restées en suspension, alors on peut y retrouver les cellules cardiogéniques révélées par la culture en méthylcellulose.

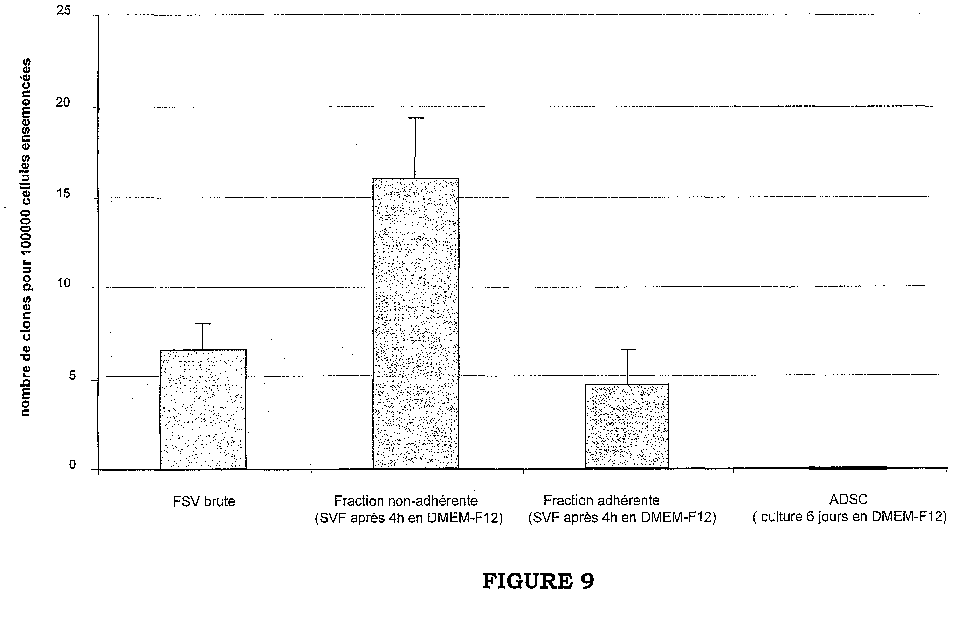

La figure 9 illustre la fréquence d'apparition des clones cardiogéniques en méthylcellulose.

- Culture des cellules de la FSV dans du milieu BHK21 uniquement :

  On observe la présence de cellules cardiogéniques.

Dans ces conditions les cellules adhèrent au support de culture cellulaire et la culture de cellules cardiogéniques est possible. Mais d'autres cellules (non cardiogéniques) adhèrent et poussent aussi, la culture est donc hétérogène.

**EXEMPLE 3 : Caractéristiques phénotypiques des populations de cellules adhérentes et non adhérentes : données *in vitro***

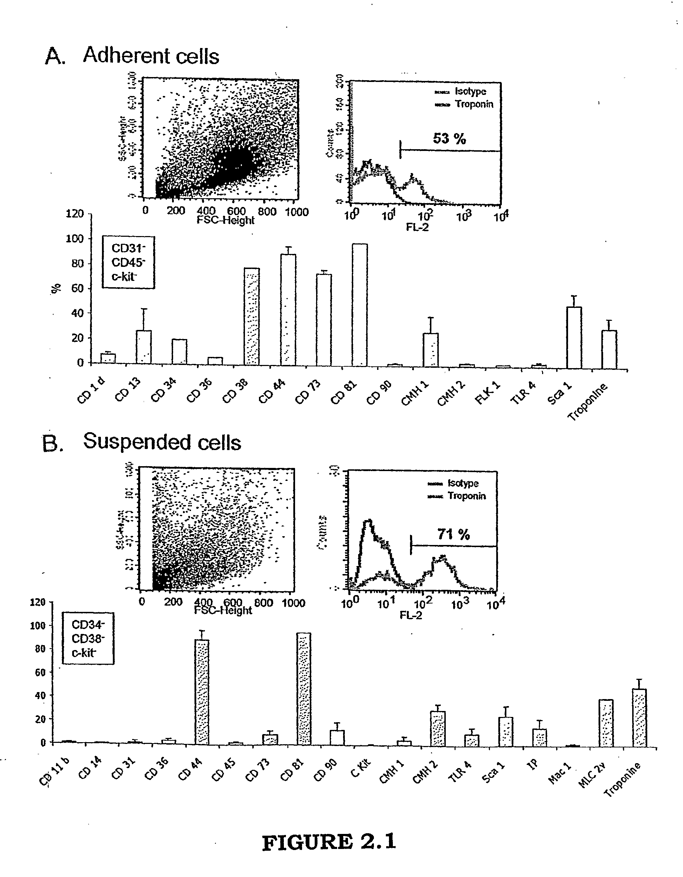

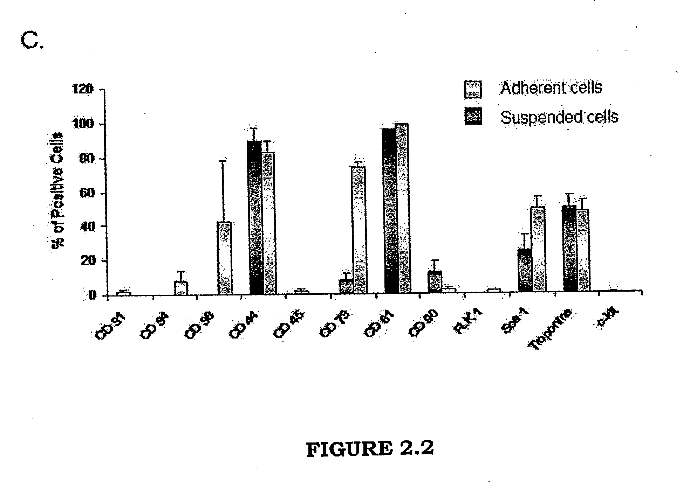

Les phénotypes cellulaires sont résumés à la figure 2. On voit que les deux types cellulaires présentent les caractéristiques phénotypiques suivantes : CD44+, CD81+, CD31-, CD45-, CD90-, ckit-, Flk1- et au moins 50 % sont positives pour Sca-1, Troponine T et MLC-2v.

La différence majeure entre les deux types cellulaires est que les cellules adhérentes sont positives pour CD38 et CD73 (74 %) et quelques unes (20 %) expriment CD34 et négatives pour CMH2, alors que ce n'est pas le cas pour les cellules non-adhérentes qui sont CMH1-.

Une expression élevée en Troponine T est inversement corrélée à une expression faible en CD90 (3-9 %).

Le Tableau IV ci-après résume ces caractéristiques.

TABLEAU IV : caractéristiques phénotypiques des cellules adhérentes et non-adhérentes

| | Cellules adhérentes (forme allongée) | Cellules en suspension (forme arrondie) |
|---|---|---|
| CD31 | - | - |
| CD34 | +/- | - |
| CD38 | + | - |
| CD44 | + | + |
| CD45 | - | - |

(suite)

|  | Cellules adhérentes (forme allongée) | Cellules en suspension (forme arrondie) |
|---|---|---|
| CD62 |  | - |
| CD73 | + | Hétérogène/- |
| CD81 | + | + |
| CD90 | Hétérogène/- | Hétérogène/- |
| C-kit | - | - |
| MLC2v | + | + (50%) |
| CMH1 | + (25-30%) | - |
| CMH2 | - | Hétérogène |
| Sca-1 | + (50%) | + (50%) |
| Troponine T | + (50%) | + (50%) |
| Flk1 | - | - |
| Expression de :<br>Brachyury<br>Islet-I<br>MEF-2c<br>Oct3/4 | +<br>+<br>+<br>+ | +<br>+<br>+<br>+ |

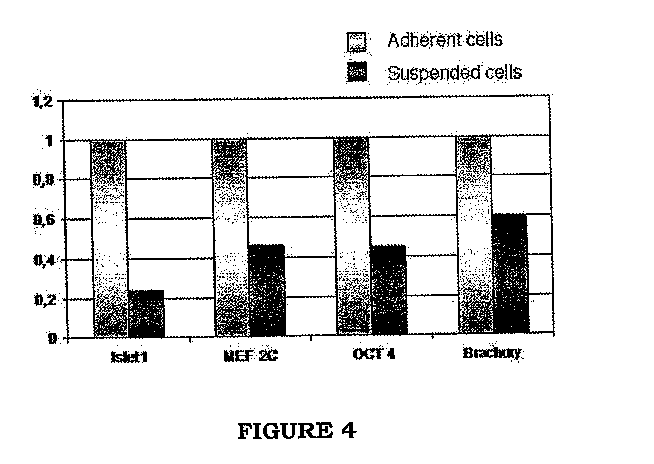

La PCR quantitative en temps réel révèle que les deux populations expriment le facteur de transcription mésodermique Brachyury, les facteurs de transcription cardiaques Islet-1 et MEF-2c, ainsi que Oct3/4, un ' facteur de liaison transcriptionnel présent dans les cellules indifférenciées ayant un potentiel de prolifération élevé (figure 4). Pour tous les transcrits, le taux d'expression est plus élevé dans les cellules adhérentes par comparaison avec les cellules non adhérentes.

Une identification concomitante de ces marqueurs traduit la présence de cellules immatures et une hétérogénéité dans le taux de différenciation de ces populations.

L'expression des protéines en fonction de la différenciation cardiaque est analysée par immunohistochimie sur les fibres adhérentes (figure 4) et montre la présence de protéines cardiaques telles que MLC-2v, titine, (3-MHC, $\alpha$-actinine sarcomérique, troponine T, même si les cellules n'expriment pas encore $\alpha$-MHC.

Ces données *in vitro* montrent que le procédé selon l'invention permet d'obtenir des quantités importantes de cellules cardiomyogéniques à partir de tissu adipeux. Les cellules en culture présentent deux phénotypes distincts qui sont néanmoins associés en culture.

La comparaison du nombre de cellules cardiomyogéniques obtenues avec le procédé selon l'invention avec le nombre de cellules obtenues avec le procédé tel que décrit dans la Demande Internationale PCT WO 02/055678 est illustrée dans le Tableau V suivant. Ces données sont obtenues à partir d'1 g de tissu adipeux ($20 \times 10^6$ cellules de FSV).

TABLEAU V

| Demande Internationale PCT WO 02/055678 | Invention |
|---|---|
| Culture en méthylcellulose<br>→14286 clones en 1 mois, soit $7.10^6$ à $14.10^6$ cellules cardiogéniques en 1 mois. Ces cellules peuvent être maintenues en milieu DMEM (pas d'expansion) | Culture en méthylcellulose + milieu BHK21<br>→$2.10^8$ cellules cardiogéniques adhérentes en 1 mois et $1,5.10^7$ cellules cardiogéniques non-adhérentes.<br>Expansion possible notamment par repiquage des cellules non-adhérentes en milieu liquide (nombreux passages) : en 8 jours, $1,5.10^7$ cellules non-adhérentes donnent $7,5.10^7$ cellules adhérentes produisant elles-mêmes $1,1.10^7$ cellules non-adhérentes tous les 2 jours pendant 4-6 semaines. |

**EXEMPLE 4 : Utilisation des cellules adhérentes et non-adhérentes selon l'exemple 1 pour induire une réparation de l'ischémie cardiaque. Différenciation *in vivo.***

Après leur injection dans un modèle d'infarctus murin (conditions de l'exemple 1.8), le devenir des cellules GFP arrondies et allongées est étudié seul ou en comparaison avec des cellules SVF-GFP brutes.

Les cellules arrondies sont obtenues par centrifugation du surnageant de culture. La culture enrichie en cellules adhérentes est collectée après trypsinisation de la couche adhérente, qui est lavée, de manière à éliminer la plupart des cellules arrondies (voir exemple 1).

Le premier groupe (n = 6) reçoit $10^5$ cellules SVF-GFP brutes (contrôle) et les coeurs sont analysés 7 ou 14 jours après l'injection.

Quel que soit le moment de l'analyse (7 ou 14 jours), on ne retrouve quasiment pas de cellules GFP chez les souris.

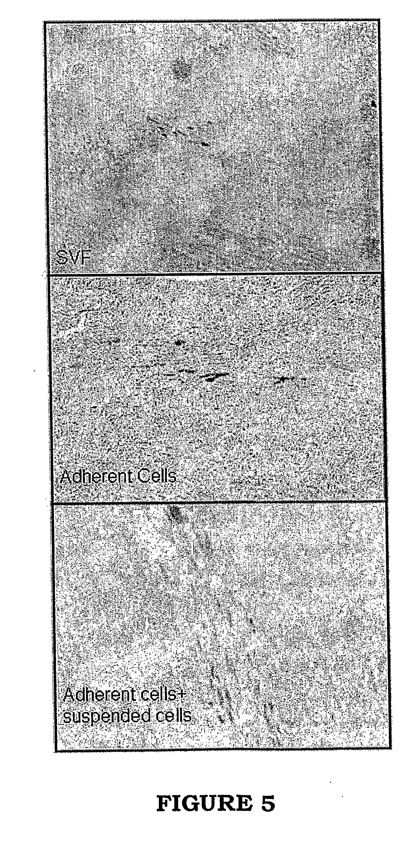

Elles présentent toujours un aspect de fibroblastes ; aucun des marqueurs cardiaques n'est détecté (figure 5).

Le deuxième groupe (n = 9) reçoit $10^5$ de cellules arrondies non adhérentes récoltées par centrifugation du milieu de culture. Chez toutes les souris de ce groupe, on ne détecte aucune cellule GFP positive à 7 jours (n = 3) ou à 14 jours (n = 2) après la transplantation.

Dans le troisième groupe (n = 7), $10^5$ cellules adhérentes enrichies sont injectées. Plus précisément, les cellules issues de la trypsination d'un tapis cellulaire obtenu selon le mode de culture MC→BHK21 ont été injectées. Bien évidemment la culture a été rincée avant l'action de la trypsine pour éliminer le maximum de cellules surnageantes. Cependant, ces dernières émanant du tapis, on est jamais sûr de tout avoir retiré par lavage, certaines cellules qui engagent des liaisons avec le tapis restent accrochées. C'est la raison pour laquelle cette population est dénommée enrichie en (presque exclusivement constituée de) cellules adhérentes.

On détecte des cellules GFP isolées dans 3 coeurs sur 4 au 7$^{eme}$ jour et dans 3 coeurs sur 5 au 14$^{eme}$ jour. La morphologie de ces cellules GFP est allongée mais pas de type cardiaque, dans la mesure où l'on n'observe aucunes stries (figure 5).

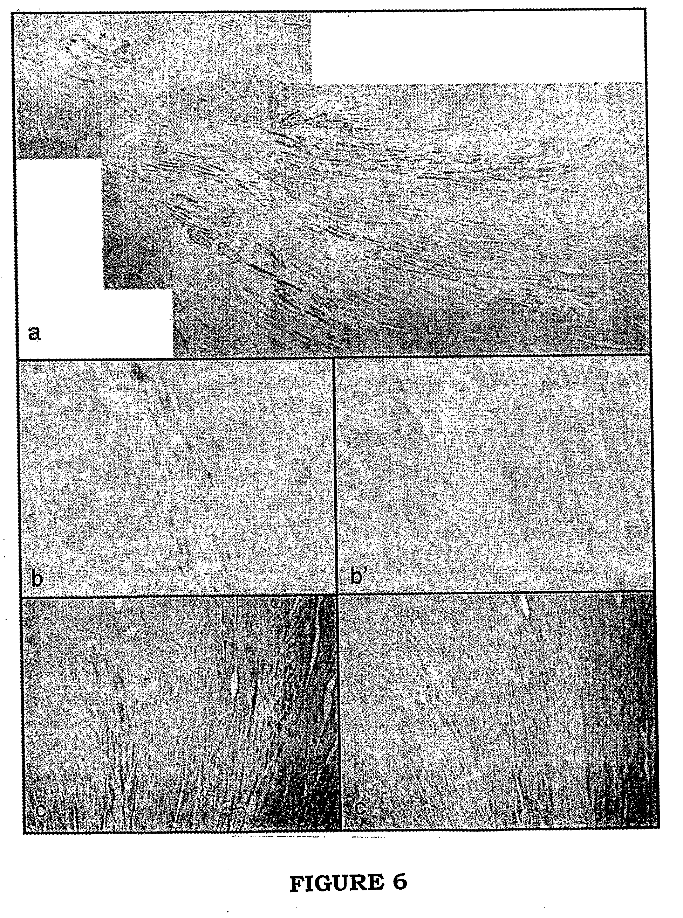

Le quatrième groupe (n = 23) reçoit un mélange 1:1 de cellules adhérentes et de cellules arrondies en suspension ($1.10^5$ cellules de cellules cardiomyogéniques expriment la GFP collectée uniquement entre le 3$^{ème}$ et le 4$^{ème}$ passage de l'étape c) ; l'injection se fait immédiatement après l'infarctus du myocarde par injection directe dans la paroi ventriculaire du mélange de cellules. Après 7 jours, les cellules GFP sont retrouvées dans tous les coeurs (11 sur 11). Un marquage GFP spécifique est observé sur les bords de la zone ischémiée, suggérant l'émergence d'une zone de régénération autour de la zone ischémiée. De plus, on observe également des cellules GFP dans la zone ischémiée. Ces cellules ont une morphologie cardiaque (figure 6) et sont dans la même orientation spatiale que le myocarde intact (figure 6). Elles sont très abondantes et forment une réelle greffe de tissu de type myocarde (figure 6). Ces cellules expriment les marqueurs de cardiomyocytes, tels que MLC-2V (figure 7A) et Troponine T (figure 7B).

Après 15 jours et un mois, ces cellules sont toujours présentes (chez 5 animaux sur 7) et conservent les mêmes caractéristiques.

Après un mois, dans 4 sur 5 des coeurs analysés, le phénotype de cellules GFP devient plus précis avec une morphologie homogène de cellules striées.

Dans le groupe dans lequel les souris reçoivent à la fois des cellules adhérentes et des cellules en suspension, on observe effectivement dans tous les coeurs, la présence de cellules GFP-positives exprimant les caractéristiques des cellules cardiaques.

Cette caractérisation immunophénotypique est renforcée par l'analyse PCR quantitative en temps réel, basée sur la présence de la séquence GFP génomique.

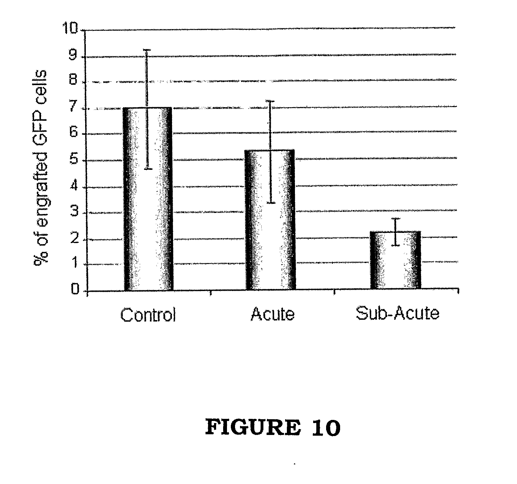

La quantité de cellules GFP injectées identifiées 7 jours après l'administration représente 5,32 % ± 1,955 dans le modèle aigu d'infarctus du myocarde, qui correspond à une moyenne de 5322 cellules GFP (figure 10).

En comparaison, dans un modèle sub-aigu d'infarctus du myocarde dans lequel les cellules sont injectées 3 jours après la ligation de la coronaire, le pourcentage de cellules GFP greffées à J7 est de 2,2 % ± 0,511 et représente une moyenne de 2201 cellules GFP (figure 10).

Une expérience contrôle, dans laquelle les souris sont sacrifiées immédiatement après l'injection cellulaire révèle que seulement 7 % des cellules injectées sont retrouvées (figure 10).

Cette quantité représente 6946 cellules parmi les 100 000 cellules injectées.

En résumé, l'injection de 100 000 cellules GFP conduit à la greffe de 7000 cellules et 7 jours plus tard 5300 à 2200 cellules GFP sont encore présentes, selon le modèle d'infarctus du myocarde impliqué.

Pour compléter l'étude et notamment l'influence de l'environnement ischémique sur la greffe, le mélange des deux populations cellulaires (cellules adhérentes et cellules non-adhérentes) sont injectées dans un infarctus du myocarde chronique (2 semaines après la ligature coronarienne).

Dans ces conditions, on n'observe pas de greffe, comme dans le modèle aigu (n = 9).

Les résultats sont résumés dans le Tableau VI.

TABLEAU VI : Résultats des greffes de cellules cardiaques selon l'invention (cellules adhérentes et cellules non-adhérentes) en mélange dans un rapport 1:1, dans différents modèles d'infarctus

| Délai entre l'injection et le sacrifice de l'animal (en jours) | Modèle aigu | Modèle sub-aigu | Modèle chronique |
|---|---|---|---|
| J7<br>J15<br>J30 | 11/11<br>5/7<br>4/5<br>cellules cardiaques<br><br>(n=23) | 2/2<br>1/1<br>5/9<br>cellules cardiaques<br><br>(n=12) | 1/5<br>0/4<br>0/5<br>absence de cellules cardiaques<br>(n=14) |

Pour discriminer l'efficacité des cellules non-adhérentes récoltées en suspension à partir du milieu de culture (n = 9) *vs* les cellules adhérentes obtenues après trypsinisation (n = 7), elles sont injectées indépendamment comme précisé ci-dessus.

De manière surprenante peu (à partir de la population de cellules non-adhérentes, figure 5) ou aucune (à partir de la population de cellules adhérentes) cellules GFP ne peuvent être identifiées 7 ou 15 jours après l'injection lorsqu'elles sont administrées séparément.

Ainsi, la coinjection des deux types cellulaires est requise.

Les résultats sont résumés dans le Tableau VII.

TABLEAU VII : Modèle aigu d'infarctus du myocarde

| Délai entre l'injection et le sacrifice de l'animal (en jours) | ADSC[(1)]<br>ADSC | Cellules cardiaques ou cardiomyogéniques selon l'invention (2) | | |
|---|---|---|---|---|
| | | Population non-adhérente | Population Adhérente | Mélange des deux populations dans un rapport 1:1 |
| J7<br>J15<br>J30 | ¾<br>2/2<br>-<br>absence de cellules cardiaques<br>(n=6) | 3/4<br>3/5<br>-<br>absence de cellules cardiaques<br>(n=9) | 0/5<br>0/2<br>-<br>absence de cellules cardiaques<br>n=7) | 11/11<br>5/7<br>4/5<br>cellules cardiaques<br><br>(n=23) |
| (1) ADSC (adipose-derived stromal cells) : cellules FSV obtenues à partir de tissu adipeux et cultivées 6 jours dans du DMEM-F12<br>(2) Cellules FSV obtenues à partir du tissu adipeux, cultivées dans de la méthylcellulose pour obtenir une différenciation cardiaque et mises en expansion dans le milieu BHK21 | | | | |

## EXEMPLE 5 : Etude fonctionnelle

Cette étude permet de vérifier si les cellules cardiaques selon l'invention protègent ou restaurent la fonction ventriculaire gauche dans le coeur ischémié.

L'évaluation échocardiographique a permis de comparer deux groupes d'animaux avec un infarctus du myocarde.

Trois jours après la chirurgie, un groupe (n = 9) reçoit $1.10^5$ cellules (mélange des deux populations de cellules), tandis que le groupe contrôle reçoit un milieu a cellulaire (n = 8).

Plusieurs paramètres ont été analysés pour estimer le remodelage (basé sur les diamètres et les volumes) et la fonction systolique (basée sur la fraction d'éjection du ventricule gauche), dans les deux groupes.

On observe aucune différence significative en ce qui concerne le volume télédiastolique du ventricule gauche (LVEDV), le volume télésystolique du ventricule gauche (LVESV) et la fraction d'éjection du ventricule gauche (LVEF), entre les deux groupes avant et 3 jours après la chirurgie.

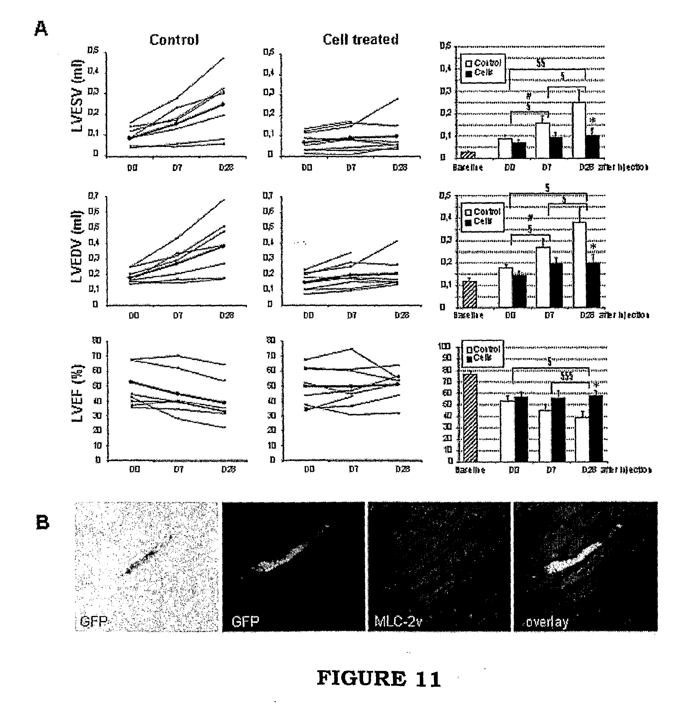

Trois jours après la ligature de l'artère coronaire et immédiatement avant l'injection (J0), la fonction cardiaque est détériorée de manière similaire dans les deux groupes (P = 0,5825) indiquant que les tailles des infarctus du myocarde étaient similaires (Figure 11).

Les animaux sont traités soit avec une injection de cellules soit avec un milieu (pour le groupe contrôle) et les paramètres cardiaques sont analysés après 7 (J7) et 28 (J28) jours.

Dans ce groupe contrôle, la LVEF est diminuée de 52,9 % ± 4,9 (J0) jusqu'à 44,7 % ± 5,8 à J7 pour finalement atteindre 38,75 % ± 5,5 à J28 (P<0,0001 J7 J28).

Dans le groupe traité avec des cellules, la LVEF reste stable (56,6 % ± 4,5 à J0 ; 56,4% ± 5,8 à J7 et 57,7 % ± 4,4 à J28), avec P = 0,967, J7 vs J28.

28 jours après l'injection la LVEF est significativement différente en faveur du groupe traité, entre le groupe traité et le groupe contrôle (P = 0,0201 contrôle traitement cellulaire).

En ce qui concerne le remodelage, une dilatation significative du ventricule gauche qui reçoit le milieu est notée après 28 jours, comme indiqué par l'augmentation significative du LVEDV de 0,18 ml ± 0,015 à J0 à 0,27 ml ± 0,039 à J7 et 0,38 ml ± 0,071 à J28 (tous les P<0,03). Dans ce groupe contrôle, une augmentation du LVESV est également observée : 0,0885 ml ± 0,015 à J0 ; 0,158 ml ± 0,032 à J7 et 0,250 ml ± 0,056 à J28 (tous les P<0,03).

Au contraire, dans les infarctus du myocarde traités avec des cellules, on n'observe aucune dilatation significative : 0,147 ml ± 0,015 à J0 ; 0,193 ml ± 0,030 à J7 ; 0,199 ml ± 0,038 à J28 pour LVEDV et 0,069 ml ± 0,013 à J0, 0,092 ml ± 0,022 à J7 et 0,100 ml ± 0,033 à J28 pour LVESV.

La comparaison du LDEDV et du LVESV après 28 jours montre une différence significative (dilatation moindre) pour le groupe traité le groupe contrôle (P = 0,046 et 0,039 respectivement).

Une performance du ventricule gauche meilleure à J28 est supportée par la présence de cellules co-exprimant GFP et MLC-2v identifiés chez 5 des 9 coeurs traités à J28 (voir figure 11).

Un pourcentage plus élevé de cellules GFP sont quantifiées dans le modèle aigu par rapport au modèle sub-aigu d'infarctus, suggérant que les cellules pouvaient être injectées trois jours après la ligation de l'artère sans conséquence sur la greffe cellulaire.

**Revendications**

1. Procédé d'obtention de cellules cardiaques, **caractérisé en ce qu'**il comprend au moins les étapes suivantes :

a) sélection des cellules cardiomyogéniques à partir de la fraction stroma-vasculaire (FSV ou SVF pour *stromal vascular fraction*) brute ;
b) culture des cellules sélectionnées à l'étape a) dans un milieu liquide optimisé pour l'expansion *ex vivo* des cellules cardiomyogéniques, lequel milieu liquide est sélectionné dans le groupe constitué par :

- le milieu BHK21 dont la composition est la suivante :

SELS INORGANIQUES, IX
liquide mg/L :

| | |
|---|---|
| $CaCl_2 \cdot 2H_2O$ | 265,00 |
| $Fe(NO_3)_3 \cdot 9H_2O$ | 0,10 |
| KCl | 400,00 |
| $MgSO_4 \cdot 7H_2O$ | 200,00 |
| NaCl | 6400,00 |
| $NaHCO_3$ | 2750,00 |
| $NaH_2PO_4 \cdot 2H_2O$ | 141,00 |

ACIDES AMINES, 1X liquide
mg/L :

| | |
|---|---|
| L-arginine $\cdot$ HCl | 42,00 |
| L-cystine | 24,00 |
| L-glutamine | 292,00 |
| L-histidine HCl $\cdot$ $H_2O$ | 21,00 |
| L-isoleucine | 52,00 |
| L-leucine | 52,00 |
| L-lysine $\cdot$ HCl | 73,00 |
| L-méthionine | 15,00 |
| L-phénylalanine | 33,00 |
| L-thréonine | 47,60 |
| L-tryptophane | 8,00 |
| L-tyrosine | 36,20 |
| L-valine | 46,80 |

ACIDES AMINES NON ESSENTIELS, 5 ml, 10 mM/500 ml,
100X liquide g/L :

| | |
|---|---|
| L-alanine | 890,00 |
| L-asparagine | 1320,00 |
| L-acide aspartique | 1330,00 |
| L-acide glutamique | 1470,00 |
| Glycine | 750,00 |
| L-proline | 1150,00 |
| L-sérine | 1050,00 |
| VITAMINES, 1X liquide mg/L : | |
| Pantothénate de calcium D | 2,00 |
| Chlorure de choline | 2,00 |
| Acide folique | 2,00 |
| i-inositol | 3,60 |

(suite)

| Nicotinamide | 2,00 |
|---|---|
| Pyridoxal HCl | 2,00 |
| Riboflavine | 0,20 |
| Thiamine HCl | 2,00 |

et contenant au moins du sérum de veau foetal et du β-mercapto-éthanol et
- tout autre milieu dont la composition en sels inorganiques, acides aminés et vitamines est identique à celle du milieu BHK21
et ledit milieu contenant au moins du sérum de veau foetal et du β-mercapto-éthanol ;

c) entretien et expansion desdites cellules par passages successifs en milieu liquide ; et
d) obtention de cellules cardiaques.

2. Procédé selon la revendication 1 (procédé 1), **caractérisé en ce que** la sélection de l'étape a) est effectuée par culture primaire de cellules de la fraction stroma-vasculaire brute dans un milieu semi-solide, jusqu'à l'émergence de clusters (ou clones) de cellules contractiles.

3. Procédé selon la revendication 2, **caractérisé en ce que** l'étape b) comprend le prélèvement desdites cellules contractiles constituées de deux sous populations présentant des types morphologiques distincts, à savoir des cellules adhérentes de type allongé et des cellules non-adhérentes de type rond (ou arrondi) ; et le repiquage desdites cellules contractiles dans ledit milieu liquide optimisé pour l'expansion *ex vivo* des cellules cardiomyogéniques ou des cellules souches.

4. Procédé selon la revendication 2 ou la revendication 3, **caractérisé en ce que** l'étape c) comprend l'entretien et l'expansion d'au moins l'une des deux sous populations de cellules (cellules adhérentes et cellules non adhérentes en suspension) par passages successifs en milieu liquide.

5. Procédé selon l'une quelconque des revendications 2 à 4, **caractérisé en ce que** le milieu semi solide de l'étape a) est avantageusement sélectionné dans le groupe constitué par les dérivés cellulosiques et les matrices membranaires basales reconstituées comprenant au moins l'un des éléments suivants : collagène, laminine et protéoglycanes.

6. Procédé selon la revendication 2, **caractérisé en ce que** la durée de culture selon l'étape a) est de quelques jours à quelques semaines, de préférence de une à deux semaines.

7. Procédé selon l'une quelconque des revendications 2 à 6, **caractérisé en ce que**, préalablement au repiquage des cellules contractiles, une seconde sélection à l'aide d'au moins un marqueur approprié est effectuée.

8. Procédé selon la revendication 7, **caractérisé en ce que** ledit marqueur est avantageusement un marqueur positif et/ou un marqueur négatif des cellules cardiaques.

9. Procédé selon la revendication 8, **caractérisé en ce que** le marqueur positif des cellules cardiaques est sélectionné dans le groupe constitué par les marqueurs suivants : Sca-1, troponine, MLC2v, CD44, CD81, CD73, CD38 et le marqueur négatif des cellules cardiaques est sélectionné dans le groupe constitué par les marqueurs du complexe majeur d'histocompatibilité CMH1 ou CMH2, CD31, CD34, CD45, c-kit et Flkl.

10. Procédé selon l'une quelconque des revendications 2 à 9, **caractérisé en ce que** ledit autre milieu liquide de l'étape b) est un milieu susceptible d'être utilisé pour des cellules de type ES.

11. Procédé selon la revendication 1 (procédé 2), **caractérisé en ce que** la sélection de l'étape a) est effectuée par tri cellulaire des cellules de la SVF brute à l'aide d'au moins un marqueur négatif des cellules cardiaques tel que défini à la revendication 9 et l'étape b) comprend la culture des cellules sélectionnées dans un milieu liquide BHK21 contenant au moins du sérum de veau foetal et du β-mercapto-éthanol ou tout autre milieu susceptible d'être utilisé pour des cellules de type ES dont la composition en sels inorganiques, acides aminés et vitamines est telle que définie à la revendication 1 et contenant au moins du sérum de veau fatal et du β-mercapto-éthanol.

**12.** Procédé selon l'une quelconque des revendications 1 à 11, **caractérisé en ce que** l'étape c) d'entretien et d'expansion des cultures en milieu liquide peuvent être réalisés selon l'une des deux modalités suivantes :

- centrifugation des cellules en suspension dans un milieu liquide identique ou différent de celui de l'étape b) et réensemencement du culot cellulaire dans le même milieu liquide ; ou
- décrochement enzymatique ou tout autre procédé permettant le détachement des cellules du tapis cellulaire adhérent, centrifugation de la suspension cellulaire décrochée et réensemencement du culot cellulaire dans le même milieu liquide.

**13.** Procédé selon l'une quelconque des revendications 1 à 12, **caractérisé en ce que** lesdites cellules cardiomyogéniques sont génétiquement modifiées.

**14.** Utilisation des cellules cardiaques, susceptibles d'être obtenues à partir des cellules de la fraction stroma-vasculaire, dans les conditions exposées dans les procédés selon l'une quelconque des revendications 1 à 13, pour la préparation d'un médicament apte à reconstituer une zone cardiaque ischémique, lesdites cellules cardiaques étant sélectionnées dans le groupe constitué par :

(i) les cellules cardiomyogéniques contractiles adhérentes de forme allongée présentant au moins les caractéristiques suivantes : $CD38^+$, $CD44^+$, $CD73^+$, $CD81^+$, $CD31^-$, $CD45^-$, Ckif, $Flk1^-$, $CMH2^-$, environ 50 % desdites cellules étant $Sca-1^+$ et troponine $T^+$, $MLC2v^+$,
(ii) les cellules non-adhérentes de forme arrondie présentant au moins les caractéristiques suivantes : $CD44^+$, $CD81^+$, $CD31^-$, $CD45^-$, $CD73^-$, $CMH1^-$, environ 50 % desdites cellules étant $Sca-1^+$, troponine $T^+$ et $MLC2v^+$, les deux types de cellules exprimant en outre le facteur de transcription mésodermique Brachyury, les facteurs de transcription Islet-1 et MEF-2c et le facteur de liaison transcriptionnel Oct3/4, ou
(iii) un mélange desdites cellules adhérentes et desdites cellules non-adhérentes.

**15.** Procédé selon l'une quelconque des revendications 1 à 13, **caractérisé en ce que** l'étape b) de culture des cellules sélectionnées en a) est effectuée sur une surface d'adhésion appropriée pour l'expansion *ex vivo* des cellules cardiomyogéniques.

**16.** Procédé selon l'une quelconque des revendications 1 à 13, **caractérisé en ce qu'**il comprend :

a) une culture primaire de cellules de la FVS dans un milieu semi-solide à base de méthylcellulose et la sélection des cellules cardiomyogéniques,
b) une culture secondaire des cellules sélectionnées en a) dans le milieu liquide BHK21 contenant au moins du sérum de veau fatal et du β-mercapto-éthanol ou tout autre milieu dont la composition en sels inorganiques, acides aminés et vitamines est telle que définie à la revendication 1 et contenant au moins du sérum de veau foetal et du (3-mercapto-éthanol sur une surface d'adhésion appropriée pour l'expansion *ex vivo* des cellules cardiomyogéniques,
c) l'entretien et l'expansion desdites cellules par passages successifs en milieu liquide et
d) l'obtention de cellules cardiaques.

**17.** Procédé selon la revendication 1, **caractérisé en ce qu'**il comprend la sélection des cellules cardiomyogéniques à partir de la fraction stroma-vasculaire (FSV) brutes fraîchement préparées par culture primaire desdites cellules de la FSV directement en milieu BHK21 et sur un support ou surface d'adhésion approprié.

**18.** Procédé selon la revendication 15 ou la revendication 17, **caractérisé en ce que** le support ou surface d'adhésion approprié est sélectionné dans le groupe constitué par la gélatine, les protéines d'adhésion et les protéines de la matrice extracellulaire.

**Patentansprüche**

**1.** Verfahren zum Erhalt von Herzzellen, **dadurch gekennzeichnet, dass** es mindestens die folgenden Schritte umfasst.:

a) Auswahl der kardiomyogenen Zellen ausgehend von der stromal-vaskulären Grobfraktion (engl. *Stromab Vascular Fraktion*, SVF')

b) Kultivierung der im Schritt a) ausgewählten Zellen in einem flüssigen Medium, das für die ex-vivo-Expansion von kardiomyogenen Zellen optimiert ist, wobei das flüssige Medium in der Gruppe ausgewählt ist, die aus Folgendem besteht:

- dem BHK21-Medium, dessen Zusammensetzung die folgende ist:

ANORGANISCHE SALZE, 1X Flüssigkeit mg/L:

| | |
|---|---|
| $CaCl_2 \cdot 2H_2O$ | 265,00 |
| $Fe(NO_3)_3 \cdot 9H_2O$ | 0,10 |
| KCl | 400,00 |
| $MgSO_4 \cdot 7H_2O$ | 200,00 |
| NaCl | 6400,00 |
| $NaHCO_3$ | 2750,00 |
| $NaH_2PO_4 \cdot 2H_2O$ | 141,00 |

AMINOSÄUREN, 1X Flüssigkeit mg/L:

| | |
|---|---|
| L-Arginin $\cdot$ HCl | 42,00 |
| L-Cystin | 24,00 |
| L-Glutamin | 292,00 |
| L-Histidin HCl $\cdot$ $H_2O$ | 21,00 |
| L-Isoleucin | 52,00 |
| L-Leucin | 52,00 |
| L-Lysin $\cdot$ HCl | 73,00 |
| L-Methionin | 15,00 |
| L-Phenylalanin | 33,00 |
| L-Threonin | 47,60 |
| L-Tryptophan | 8,00 |
| L-Tyrosin | 36,20 |
| L-Valin | 46,80 |

NICHT ESSENTIELLE AMINOSÄUREN, 5 ml, 10 mM/500 ml, 100X Flüssigkeit g/L:

| | |
|---|---|
| L-Alanin | 890,00 |
| L-Asparagin | 1320,00 |
| L-Asparaginsäure | 1330,00 |
| L-Glutaminsäure | 1470,00 |
| Glycin | 750,00 |
| L-Prolin | 1150,00 |
| L-Serin | 1050,00 |

VITAMINE, 1X Flüssigkeit mg/L:

| | |
|---|---|
| D-Calciumpantothenat | 2,00 |
| Cholinchlorid | 2,00 |
| Folsäure | 2,00 |
| I-Inositol | 3,60 |
| Nicotinamid | 2,00 |
| Pyridoxal-HCl | 2,00 |
| Riboflavin | 0,20 |
| Thiamin-HCl | 2,00 |

EP 1 984 490 B1

und mindestens ein fötales Kälberserum und β-Mercaptoethanol enthält und

- jedem anderen Medium, dessen Zusammensetzung an anorganischen Salzen, Aminosäuren und Vitaminen identisch mit derjenigen des BHK21-Mediums ist,
und das Medium mindestens fötales Kälberserum und β-Mercaptoethanol enthält;

c) Haltung und Expansion der Zellen durch aufeinanderfolgende Durchgänge im flüssigen Medium; und
d) Erhalt von Herzzellen.

2. Verfahren nach Anspruch 1 (Verfahren 1), **dadurch gekennzeichnet, dass** die Auswahl des Schrittes a) durch Primärkultur von Zellen der stromal vaskulären Grobfraktion in einem halbfesten Medium bis zum Auftreten von Clustern (oder Klonen) von kontraktilen Zellen durchgeführt wird.

3. Verfahren nach Anspruch 2, **dadurch gekennzeichnet, dass** der Schritt b) die Entnahme der kontraktilen Zellen, die aus zwei Unterpopulationen bestehen, die unterschiedliche morphologische Typen aufweisen, nämlich adhärente Zellen vom länglichen Typ und nicht adhärente Zellen vom runden (oder rundlichen) Typ; und die Passage der kontraktilen Zellen in das für die *ex-vivo*-Expansion der kardiomyogenen Zellen oder der Stammzellen optimierte flüssige Medium umfasst.

4. Verfahren nach Anspruch 2 oder Anspruch 3, **dadurch gekennzeichnet, dass** der Schritt c) die Haltung und die Expansion von mindestens einer der zwei Zellunterpopulationen (adhärente Zellen und nicht adhärente Suspensionszellen) durch aufeinanderfolgende Durchgänge in dem flüssigen Medium umfasst.

5. Verfahren nach einem der Ansprüche 2 bis 4, **dadurch gekennzeichnet, dass** das halbfeste Medium von Schritt a) vorteilhafterweise in der Gruppe ausgewählt wird, die aus Cellulosederivaten und rekonstituierten Basalmembranmatrizen besteht, die mindestens eines der folgenden Elemente umfassen: Collagen, Laminin und Proteoglykane.

6. Verfahren nach Anspruch 2, **dadurch gekennzeichnet, dass** die Kultivierungsdauer nach Schritt a) einige Tage bis einige Wochen, vorzugsweise ein bis zwei Wochen, beträgt.

7. Verfahren nach einem der Ansprüche 2 bis 6, **dadurch gekennzeichnet, dass** vor der Passage der kontraktilen Zellen eine zweite Auswahl mittels mindestens eines zweckmäßigen Markers durchgeführt wird.

8. Verfahren nach Anspruch 7, **dadurch gekennzeichnet, dass** der Marker vorzugsweise ein positiver Marker und/oder ein negativer Marker der Herzzellen ist.

9. Verfahren nach Anspruch 8, **dadurch gekennzeichnet, dass** der positiven Marker der Herzzellen in der Gruppe ausgewählt wird, die aus den folgenden Markern besteht: Sca-1, Troponin, MLC2v, CD44, CD81, CD73, CD38, und der negative Marker der Herzzellen in der Gruppe ausgewählt wird, die aus den Markern des Haupthistokompatibilitätskomplexes CMH1 oder CMH2, CD31, CD34, CD45, c-kit und Flk1 besteht.

10. Verfahren nach einem der Ansprüche 2 bis 9, **dadurch gekennzeichnet, dass** das andere flüssige Medium von Schritt b) ein Medium ist, das für Zellen vom Typ ES verwendet werden kann.

11. Verfahren nach Anspruch 1 (Verfahren 2), **dadurch gekennzeichnet, dass** die Auswahl von Schritt a) durch Zellsortierung der Zellen der Grob-SVF mittels mindestens eines negativen Markers der Herzzellen, wie Anspruch 9 definiert, durchgeführt wird und der Schritt b) die Kultivierung der ausgewählten Zellen in einem flüssigen BHK21-Medium, das mindestens ein fötales Kälberserum und β-Mercaptoethanol enthält, oder jedem anderen Medium umfasst, das für Zellen vom Typ ES verwendet werden kann, dessen Zusammensetzung an anorganischen Salzen, Aminosäuren und Vitaminen ist, wie in Anspruch 1 definiert, und mindestens fötales Kälberserum und β-Mercaptoethanol enthält.

12. Verfahren nach einem der Ansprüche 1 bis 11, **dadurch gekennzeichnet, dass** der Schritt c) der Haltung und Expansion der Kulturen im flüssigen Medium nach einer der zwei folgenden Arten ausgeführt werden kann:

- Zentrifugierung der Suspensionszellen in einem flüssigen Medium, das mit demjenigen von Schritt b) identisch ist oder sich davon unterscheidet, und erneutes Aussäen des Zellpellets in dem gleichen flüssigen Medium; oder
- enzymatische Trennung oder jedes andere Verfahren, das die Ablösung der Zellen vom adhärenten Zellrasen ermöglicht, Zentrifugierung der getrennten Zellsuspension und erneutes Aussäen des Zellpellets in dem gleichen flüssigen Medium.

**13.** Verfahren nach einem der Ansprüche 1 bis 12, **dadurch gekennzeichnet, dass** die kardiomyogenen Zellen genetisch verändert sind.

**14.** Verwendung von Herzzellen, die ausgehend von Zellen der stromal vaskulären Fraktion erhalten werden können, in den Bedingungen, die in den Verfahren nach einem der Ansprüche 1 bis 13 dargelegt sind, zur Herstellung eines Medikaments, das geeignet ist, eine ischämische Herzzone wiederherzustellen, wobei die Herzzellen in der Gruppe ausgewählt werden, die aus Folgendem besteht:

(i) den adhärenten kontraktilen kardiomyogenen Zellen mit länglicher Form, die mindestens die folgenden Eigenschaften aufweisen: $CD38^+$, $CD44^+$, $CD73^+$, $CD81^+$, $CD31^-$, $CD45^-$, $Ckit^-$, $Flk1$, $CMH2^-$, wobei ungefähr 50% der Zellen $Sca-1^+$ und Troponin $T^+$, $MLC2v^+$ sind,
(ii) den nicht adhärenten Zellen mit rundlicher Form, die mindestens die folgenden Eigenschaften aufweisen: $CD44^+$, $CD81^+$, $CD31^-$, $CD45^-$, $CD73^-$, $CMH1^-$, wobei ungefähr 50% der Zellen $Sca-1^+$ und Troponin $T^+$, $MLC2v^+$ sind,
wobei die zwei Typen von Zellen ferner den mesodermalen Transkriptionsfaktor Brachyury, die Transkriptionsfaktoren Islet-1 und MEF-2c und den transkriptionellen Bindungsfaktor Oct3/4 exprimieren, oder
(iii) einer Mischung der adhärenten Zellen und der nicht adhärenten Zellen.

**15.** Verfahren nach einem der Ansprüche 1 bis 13, **dadurch gekennzeichnet, dass** der Schritt b) zur Kultivierung der in a) ausgewählten Zellen auf einer Adhäsionsfläche durchgeführt wird, die für die *ex-vivo*-Expansion der kardiomyogenen Zellen zweckmäßig ist.

**16.** Verfahren nach einem der Ansprüche 1 bis 13, **dadurch gekennzeichnet, dass** es Folgendes umfasst:

a) eine Primärkultur mit Zellen von der SVF in einem halbfesten Medium auf Methylcellulosebasis und die Auswahl von kardiomyogenen Zellen,
b) eine Sekundärkultur aus in a) ausgewählten Zellen in dem flüssigen BHK21-Medium, das mindestens fötales Kälberserum und β-Mercaptoethanol enthält, oder jedem anderen Medium, dessen Zusammensetzung an anorganischen Salzen, Aminosäuren und Vitaminen derart ist, wie in Anspruch 1 definiert, und mindestens ein fötales Kälberserum und β-Mercaptoethanol auf einer zweckmäßigen Adhäsionsfläche für die *ex-vivo*-Expansion der kardiomyogenen Zellen enthält,
c) Haltung und Expansion der Zellen durch aufeinanderfolgende Durchgänge in dem flüssigen Medium und
d) Erhalt von Herzzellen.

**17.** Verfahren nach Anspruch 1, **dadurch gekennzeichnet, dass** es die Auswahl von kardiomyogenen Zellen ausgehend von der stromal vaskulären Grobfraktion (SVF) umfasst, die frisch durch die Primärkultur der Zellen der SVF direkt in dem BHK21-Medium und auf einem/einer zweckmäßigen Adhäsionsträger oder - fläche vorbereitet wurden.

**18.** Verfahren nach Anspruch 15 oder Anspruch 17, **dadurch gekennzeichnet, dass** der/die zweckmäßige Adhäsionsträger oder -fläche in der Gruppe ausgewählt wird, die aus Gelatine, Adhäsionsproteinen und Proteinen der extrazellulären Matrix besteht.

### Claims

**1.** A method to obtain cardiac cells, **characterized in that** it comprises at least the following steps:

a) selecting cardiomyogenic cells from the crude stromal vascular fraction (SVF);
b) culturing the cells selected at step a) in a liquid medium optimised for *ex vivo* expansion of cardiomyogenic cells, said liquid medium being selected from the group consisting of:

- BHK21 medium having the following composition:

INORGANIC SALTS, 1X liquid mg/L:

| | |
|---|---|
| $CaCl_2 \cdot 2H_2O$ | 265.00 |
| $Fe(NO_3)_3 \cdot 9H_2O$ | 0.10 |
| KCl | 400.00 |
| $MgSO_4 \cdot 7H_2O$ | 200.00 |
| NaCl | 6400.00 |
| $NaHCO_3$ | 2750.00 |
| $NaH_2PO_4 \cdot 2H_2O$ | 141.00 |

AMINO ACIDS, 1X liquid mg/L:

| | |
|---|---|
| L-arginine $\cdot$ HCl | 42.00 |
| L-cysteine | 24.00 |
| L-glutamine | 292.00 |
| L-histidine HCl $\cdot$ $H_2O$ | 21.00 |
| L-isoleucine | 52.00 |
| L-leucine | 52.00 |
| L-lysine $\cdot$ HCl | 73.00 |
| L-methionine | 15.00 |
| L-phenylalanine | 33.00 |
| L-threonine | 47.60 |
| L-tryptophan | 8.00 |
| L-tyrosine | 36.20 |
| L-valine | 46.80 |

NON-ESSENTIAL AMINO ACIDS, 5 ml, 10 mM/500ml, 100X liquid q/L:

| | |
|---|---|
| L-alanine | 890.00 |
| L-asparagine | 1320.00 |
| L-aspartic acid | 1330.00 |
| L-glutamic acid | 1470.00 |
| Glycine | 750.00 |
| L-proline | 1150.00 |
| L-serine | 1050.00 |

VITAMINS, 1X liquid mg/L:

| | |
|---|---|
| D Calcium pantothenate | 2.00 |
| Choline chloride | 2.00 |
| Folic acid | 2.00 |
| i-inositol | 3.60 |
| Nicotinamide | 2.00 |
| Pyridoxal HCl | 2.00 |
| Riboflavin | 0.20 |
| Thiamine HCl | 2.00 |

and containing at least foetal calf serum and ß-mercaptoethanol; and

- any other medium having the same composition of inorganic salts, amino acids and vitamins as the BHK21 medium,
and said medium containing at least foetal calf serum and ß-mercaptoethanol;

c) maintaining and expanding said cells via successive passages in liquid medium; and

d) obtaining cardiac cells;

2. A method according to claim 1 (method 1), **characterized in that** the selection at step a) is performed by primary culture of crude stromal vascular fraction cells in a semi-solid medium, until emergence of clusters (or clones) of contractile cells.

3. The method according to claim 2, **characterized in that** step b) comprises the harvesting of said contractile cells formed of two sub-populations having different morphological types, namely adherent cells of elongate type and non-adherent cells of round (or rounded type); and passaging said contractile cell in said optimised liquid medium for *in vivo* expansion of the cardiomyogenic cells or stem cells.

4. The method according to claim 2 or claim 3, **characterized in that** step c) comprises the maintaining and expanding of at least one of the two cell sub-populations (adherent cells and non-adherent cells in suspension) via successive passaging in liquid medium.

5. The method according to any of claims 2 to 4, **characterized in that** the semi-solid medium of step a) is advantageously selected from the group consisting of cellulose derivatives and reconstituted basement membrane matrices comprising at least one of the following elements: collagen, laminin and proteoglycans.

6. The method according to claim 2, **characterized in that** the culture time at step a) is several days to several weeks, preferably one to two weeks.

7. The method according to any of claims 2 to 6, **characterized in that**, prior to passaging the contractile cells, a second selection is performed using at least one appropriate marker.

8. The method according to claim 7, **characterized in that** said marker is advantageously a positive marker and/or negative marker of cardiac cells.

9. The method according to claim 8, **characterized in that** the positive marker of cardiac cells is selected from the group consisting of the following markers: Sca-1, troponin, MLC2v, CD44, CD81, CD73, CD38, and the negative marker of the cardiac cells is selected from the group consisting of markers of the major histocompatibility complex MHC I or MHC II, CD31, CD34, CD45, c-kit and Flk1.

10. The method according to any of claims 2 to 9, **characterized in that** said other liquid medium at step b) is a medium able to be used for ES-type cells.

11. A method according to claim 1 (method 2), **characterized in that** the selection at step a) is performed via cell sorting of crude SVF cells by means of at least one negative marker of cardiac cells such as defined in claim 9, and step b) comprises culturing of the selected cells in liquid BHK21 medium containing at least foetal calf serum and ß-mercaptoethanol or any other medium able to be used for ES-type cells having a composition of inorganic salts, amino acids and vitamins such as defined in claim 1 and containing at least foetal calf serum and ß-mercaptoethanol.

12. The method according to any of claims 1 to 11, **characterized in that** step c) to maintain and expand cultures in a liquid medium can be performed in accordance with one of the following two modalities:

- centrifuging the cells in suspension in a liquid medium the same as or different from the medium of step b) and re-seeding the cell pellet in the same liquid medium; or
- enzymatic detachment or any other method allowing the cells to be detached from the adherent cell layer, centrifugation of the detached cell suspension and reseeding of the cell pellet in the same liquid medium.

13. The method according to any of claims 1 to 12, **characterized in that** said cardiomyogenic cells are genetically modified.

14. The use of cardiac cells able to be obtained from stromal vascular fraction cells under the conditions set forth in the methods according to any of claims 1 to 13, to prepare a medicinal product capable of reconstituting an ischaemic cardiac region, said cardiac cells being selected from the group consisting of:

(i) adherent contractile cardiomyogenic cells of elongate shape having at least the following characteristics:

CD38$^+$, CD44$^+$, CD73$^+$, CD81$^+$, CD31$^-$, CD45$^-$, Ckit$^-$, Flkl$^-$, MHC-II$^-$, about 50 % of said cells being Sca-1$^+$ and troponin T$^+$, MLC2v$^+$;

(ii) non-adherent cells of rounded shape having at least the following characteristics: CD44$^+$, CD81$^+$, CD31$^-$, CD45$^-$, CD73$^-$, MHC-I$^-$, about 50 % of said cells being Sca-1$^+$, troponin T$^+$ and MLC2v$^+$;

both types of cells also expressing the Brachyury mesodermal transcription factor, Islet-1 and MEF2C transcription factors and the Oct-3/4 binding transcription factor, or

iii) a mixture of said adherent cells and said non-adherent cells.

**15.** The method according to any of claims 1 to 13, **characterized in that** culture step b) of the cells selected at a) is performed on an adhesion surface suitable for ex *vivo* expansion of cardiomyogenic cells.

**16.** The method according to any of claims 1 to 13, **characterized in that** it comprises:

a) primary culture of SVF cells in a semi-solid methylcellulose medium and selection of the cardiomyogenic cells;
b) secondary culture of the cells selected at a) in BHK21 liquid medium containing at least foetal calf serum and ß-mercaptoethanol, or in any other medium having a composition of inorganic salts, amino acids and vitamins such as defined in claim 1 and containing at least foetal calf serum and ß-mercaptoethanol, on an adhesion surface suitable for ex *vivo* expansion of cardiomyogenic cells;
c) maintaining and expanding said cells via successive passaging in liquid medium; and
d) obtaining cardiac cells.

**17.** The method according to claim 1, **characterized in that** it comprises the selection of cardiomyogenic cells from the crude stromal vascular fraction SVF cells freshly prepared by primary culture of said SVF cells directly in BHK21 medium and on a suitable adhesion substrate or surface.

**18.** The method according to claim 15 or claim 17, **characterized in that** the suitable adhesion substrate or surface is selected from the group consisting of gelatine, adhesion proteins and extracellular matrix proteins.

A.

| Methylcellulose | BHK21 | (Methylcellulose →) BHK21 |

B.

| Culture medium | DMEM-F12 | BHK21 | MC->BHK21 |
|---|---|---|---|
| Troponine T + | 0% | 10,5% | 57% |

O number of cells / cm²
● number of cells Troponin T+/ cm²

**FIGURE 1**

## A. Adherent cells

## B. Suspended cells

**FIGURE 2.1**

C.

**FIGURE 2.2**

**FIGURE 3**

FIGURE 4

**FIGURE 5**

**FIGURE 6**

GFP

MLC-2v

Overlay

GFP

MLC-2v

Overlay

**FIGURE 7**

GFP                     Troponin T / HE

**FIGURE 8**

FIGURE 9

**FIGURE 10**

**FIGURE 11**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 02055678 A

- US 20030082152 A

**Littérature non-brevet citée dans la description**

- **WOLLERT et al.** *Circulation,* 2005, vol. 112 (2), 151-153
- **KEHAT et al.** *J. Clin. Invest.,* 2001, vol. 108, 407-444
- **MULLER et al.** *FASEB J.,* 2000, vol. 14, 2540-2558
- **TOMA et al.** *Circulation,* 2002, vol. 105, 93-98
- **LIECHTY et al.** *Nat. Medecine,* 2000, vol. 11, 1282-1286
- **CONDERELLI et al.** *PNAS,* 2001, vol. 98, 10733-10738
- **WANG et al.** *J. Thorac. Cardiovasc. Surg,* 2001, vol. 122, 699-705
- **JACKSON et al.** *J. Clin. Invest.,* 2001, vol. 107, 1395-1402
- *M/S,* 2004, vol. 6-7 (20), 651-661
- **PLANAT et al.** *Cire. Res.,* 2004, vol. 94, 223-229
- **PLANAT-BÉNARD et al.** *Circ. Res.,* 2004, vol. 94, 223-229
- **PLANAT-BÉNARD V. et al.** *Circulation,* 2004, vol. 109, 656-663
- **ZUK PA et al.** *Mol. Biol. Cell,* 2002, vol. 13, 4279-95
- **ERICKSON GR et al.** *Biochem. Biophys. Res. Commun.,* 2002, vol. 290, 763-9
- **COUSIN B. et al.** *Biochem. Biophys. Res. Commun.,* 2003, vol. 301, 1016-22
- **SAFFORD KM et al.** *Biochem. Biophys. Res. Commun.,* 2002, vol. 294, 371-9
- **BJÖRNTORP et al.** *J. Lipid. Res.,* 1978, vol. 19, 316-24
- **POITRAS et al.** *Reviews in Biology and Biotechnology,* 2002, vol. 2, 1-11
- **HEID C. et al.** *Genome Research,* 1996, vol. 6, 986-994
- **GIBSON U. et al.** *Genome Research,* 1996, vol. 6, 995-1001
- **PFAFFL et al.** *Nucleic Acids Res.,* 2002, vol. 30 (9), e36
- **OKABE M. et al.** *FEBS Lett.,* 1997, vol. 407 (3), 313-319